(19) Europäisches Patentamt
European Patent Office
Office européen des brevets

(11) **EP 3 354 188 A1**

(12) **EUROPEAN PATENT APPLICATION**
published in accordance with Art. 153(4) EPC

(43) Date of publication:
**01.08.2018 Bulletin 2018/31**

(51) Int Cl.:
***A61B 1/00*** *(2006.01)*          ***A61B 1/06*** *(2006.01)*

(21) Application number: **15904712.5**

(86) International application number:
**PCT/JP2015/076930**

(22) Date of filing: **24.09.2015**

(87) International publication number:
**WO 2017/051455 (30.03.2017 Gazette 2017/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(71) Applicant: **Olympus Corporation
Shibuya-ku
Tokyo 151-0072 (JP)**

(72) Inventors:
• **DAIDOJI, Bakusui
Tokyo 192-8507 (JP)**
• **ITO, Takeshi
Tokyo 192-8507 (JP)**

(74) Representative: **Winter, Brandl, Fürniss, Hübner,
Röss, Kaiser, Polte - Partnerschaft mbB
Patent- und Rechtsanwaltskanzlei
Alois-Steinecker-Strasse 22
85354 Freising (DE)**

(54) **ENDOSCOPE DEVICE**

(57)     An endoscope apparatus (10) includes an imager (22) that detects reflected and scattered light of illumination light radiated to an observation object to output an imaging signal, and an image processor (24) that generates an image signal from the imaging signal. The image processor (24) generates an emphasis image signal corresponding to narrow band light included in an emphasis wavelength range that includes, for an optical absorption spectrum of a diagnosis target substance present in the observation object (O), at least one of at least a maximum wavelength that takes at least a maximum value and a color-range largest wavelength that takes a color-range largest value that is a largest value of the optical absorption spectrum in any one color range of three color ranges, a first color range, a second color range, and a third color range, and a non-emphasis image signal corresponding to narrow band light included in a non-emphasis wavelength range that is a wavelength range that does not include the emphasis wavelength range.

F I G. 1

EP 3 354 188 A1

**Description**

FIELD

The present invention relates to an endoscope apparatus capable of highlighting a diagnosis target substance present in an observation object.

BACKGROUND

For example, Jpn. Pat. Appln. KOKAI No. 2014-61152 (hereinafter referred to as patent literature 1) discloses an endoscope apparatus capable of highlighting a blood vessel of an observation object. This endoscope apparatus is provided with a blood vessel emphasis filters that allow transmission of wavelength ranges of 405 to 425 nm and 530 to 550 nm for broadband light, which have a high absorption coefficient for hemoglobin, the diagnosis target substance present in the observation target, and which are used as blood vessel emphasis illumination light. Of the blood vessel emphasis illumination light that is illumination light transmitted through the blood vessel emphasis filters, the blue narrow band light of 405 to 425 nm serves to obtain an image signal in which superficial blood vessels have a high contrast and the green narrow band light of 530 to 550 nm serves to obtain an image signal in which middle-deep blood vessels have a high contrast.

Therefore, the blue narrow band light of 405 to 425 nm and the green narrow band light of 530 to 550 nm enable the superficial blood vessels and the middle-deep blood vessels to be highlighted.

CITATION LIST

[Patent Literature]

Patent Literature 1: Jpn. Pat. Appln. KOKAI Publication No. 2014-61152

SUMMARY

TECHNICAL PROBLEM

In the endoscope apparatus disclosed in the above-mentioned patent literature 1, however, when, for example, the superficial blood vessels should be observed in detail, the middle-deep blood vessels that are also highlighted may hinder the observation of the superficial blood vessels. Conversely, when the deep blood vessels should be observed in detail, the superficial blood vessels that are also highlighted may hinder the observation of the middle-deep blood vessels.

An object of the present invention, which has been made in view of the above points, is to provide an endoscope apparatus capable of highlighting a diagnosis target substance in a specific depth region relatively.

SOLUTION TO PROBLEM

An aspect of an endoscope apparatus according to the present invention includes an imager that detects reflected and scattered light of illumination light radiated to an observation object to output an imaging signal, and an image processor that generates an image signal from the imaging signal. The image processor generates an emphasis image signal corresponding to narrow band light included in an emphasis wavelength range that includes, for an optical absorption spectrum of a diagnosis target substance present in the observation object, at least one of at least a maximum wavelength that takes at least a maximum value and a color-range largest wavelength that takes a color-range largest value that is a largest value of the optical absorption spectrum in any one color range of three color ranges, a first color range, a second color range, and a third color range, and a non-emphasis image signal corresponding to narrow band light included in a non-emphasis wavelength range that is a wavelength range that does not include the emphasis wavelength range.

ADVANTAGEOUS EFFECTS OF INVENTION

According to the present invention, an emphasis image signal and a non-emphasis image signal are generated, so that, by displaying the emphasis image signal and non-emphasis image signal, a diagnosis target substance located in a specific depth region can be relatively highlighted.

BRIEF DESCRIPTION OF THE DRAWINGS

FIG. 1 is a block diagram showing a schematic structure of an endoscope apparatus according to a first embodiment.

FIG. 2 is an outside diagram showing a schematic structure of the endoscope apparatus.

FIG. 3 is a diagram showing an optical absorption spectrum of oxyhemoglobin.

FIG. 4 is a diagram showing an example of spectroscopic characteristics of color filters of an imager.

FIG. 5 is a diagram showing how a maximum value and a color-range largest value are in each color range.

FIG. 6A is a diagram schematically showing a laminated structure of blood vessels.

FIG. 6B is a diagram schematically showing how the reach length of light is in each color range.

FIG. 7 is a table showing how laser light sources to be turned on are combined in each observation mode.

FIG. 8 is a table showing how rays of laser light to be radiated are combined in each observation mode.

FIG. 9 is a schematic diagram showing a light converter.

FIG. 10 is a diagram showing how an illumination light spectrum is in a superficial blood vessel emphasis mode.

FIG. 11 is a diagram showing an example of an observation object image displayed in the superficial blood vessel emphasis mode.

FIG. 12 is a diagram showing how an illumination light spectrum is in an intermediate blood vessel emphasis mode.

FIG. 13 is a diagram showing an example of an observation object image displayed in the intermediate blood vessel emphasis mode.

FIG. 14 is a diagram showing how an illumination light spectrum is in a deep blood vessel emphasis mode.

FIG. 15 is a diagram showing an example of an observation object image displayed in the deep blood vessel emphasis mode.

FIG. 16 is a diagram showing how an illumination light spectrum is in a normal observation mode.

FIG. 17 is a diagram showing an example of how the illumination light spectrum is in a superficial blood vessel emphasis mode according to modification 1.

FIG. 18 is a diagram showing another example of how the illumination light spectrum is in the superficial blood vessel emphasis mode according to modification 1.

FIG. 19 is a diagram showing still another example of how the illumination light spectrum is in the superficial blood vessel emphasis mode according to modification 1.

FIG. 20 is a diagram showing another example of how the illumination light spectrum is in the superficial blood vessel emphasis mode according to modification 1.

FIG. 21 is a table showing an example of how laser light source lighting timing/image signal acquisition is according to modification 2.

FIG. 22 is a table showing another example of how laser light source lighting timing/image signal acquisition is according to modification 2.

FIG. 23 is a diagram showing a structure of an image processor of an endoscope apparatus according to a second embodiment of the present invention.

FIG. 24A is a diagram showing an example of illumination light including broadband light.

FIG. 24B is a diagram showing another example of illumination light including broadband light.

FIG. 25 is a diagram showing spectroscopic characteristics used for estimating one emphasis image signal and two non-emphasis image signals from an image signal of broadband light.

FIG. 26 is a diagram showing how an optical absorption spectrum of reduced hemoglobin is according to modification 4.

DESCRIPTION OF EMBODIMENTS

Hereinafter, a description will be given of embodiments of the present invention.

[First Embodiment]

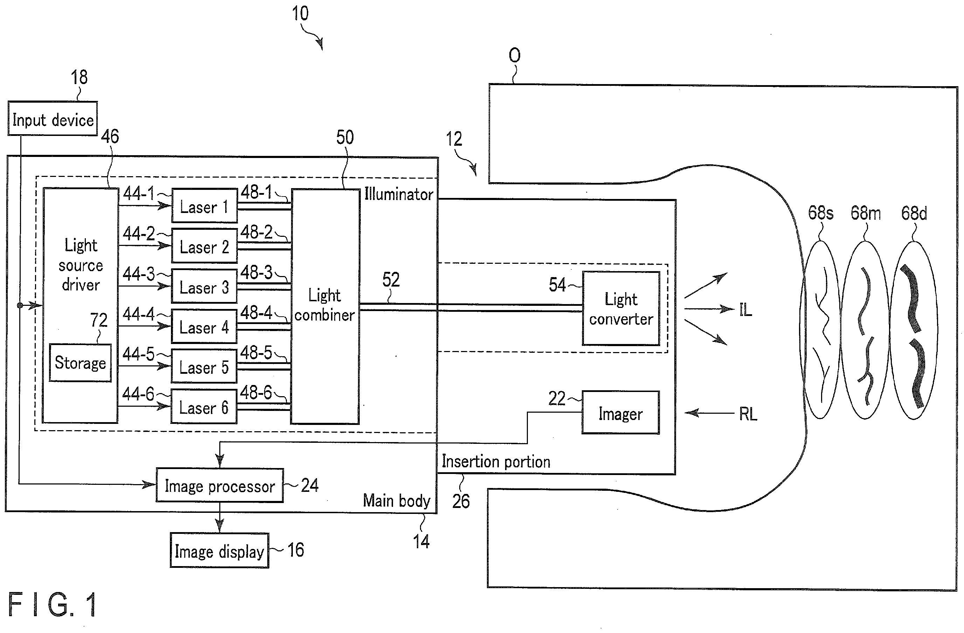

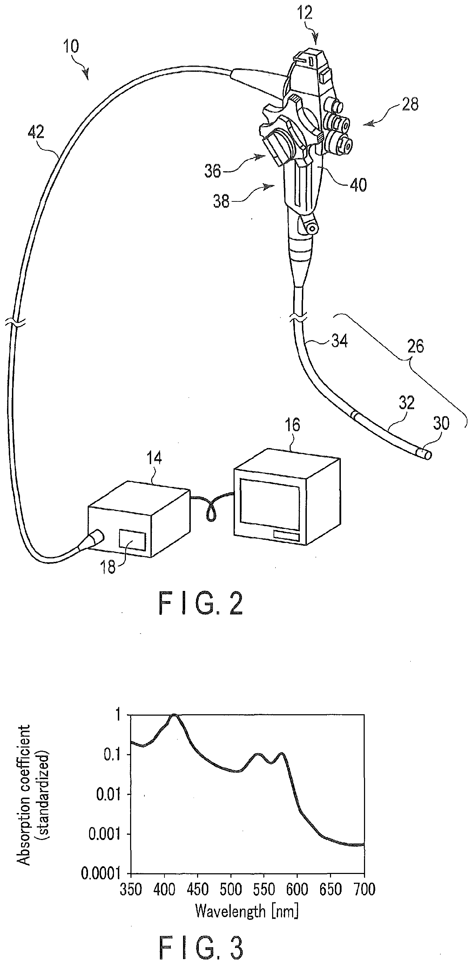

FIG. 1 and FIG. 2 are diagrams showing a schematic structure of an endoscope apparatus 10 according to a first embodiment. In the present specification, the endoscope is not limited to a medical endoscope (an esophagogastroduodenoscope, a colonoscopy, an ultrasonic endoscope, a cystoscope, a pyeloscope, a bronchoscope, or the like) or an industrial endoscope, but refers to a general type of apparatus having an insertion section to be inserted into an observation object O.

In the following, a medical endoscope will be described as an example of the endoscope.

The endoscope apparatus 10 according to the present embodiment includes an endoscope 12, a main body (video processor) 14, an image display (monitor) 16, and an input device 18. The endoscope 12 and the main body 14 are provided with an illuminator 20 that radiates illumination light IL to the observation object O. The observation object O is, for example, an affected portion or a disease portion in a subject (e.g., a body cavity (lumen)).

The endoscope 12 includes an imager 22 that detects reflected and scattered light RL of illumination light radiated to the observation object O to output an imaging signal. The main body 14 includes an image processor 24 that generates an image signal from the imaging signal of the imager 22 of the endoscope 12. The image display 16 is connected to the main body 14 and displays an observation object image formed by the image signal generated by the image processor 24. The input device 18 is connected to the main body 14 or is arranged on the main body 14, and allows various user instructions, such as the designation of an observation mode to be detailed later, to the main body 14 to be entered.

The endoscope 12 has a thin and long insertion section 26, which is a bendable member, and a handling section 28 coupled to the proximal end of the insertion section 26. The endoscope 12 is a tubular insertion apparatus having its tubular insertion section 26 to be inserted into the body cavity.

The insertion section 26 includes, from its distal end to its proximal end, a distal end hard section 30, a bendable section 32, and a flexible tube section 34. The proximal end of the distal end hard section 30 is coupled to the distal end of the bendable section 32, and the proximal end of the bendable section 32 is coupled to the distal end of the flexible tube section 34.

The distal end hard section 30 is a distal end section of the insertion section 26 and is also a distal end section of the endoscope 12, and is a hard member. The distal end hard section 30 is provided with the imager 22.

The bendable section 32 can be bent in a desirable direction in accordance with an operation by the user (the operator such as a medical doctor) through a bending operation section 36 provided at the handling section 28. The user causes the bendable section 32 to be bent by operating the bending operation section 36. The position and direction of the distal end hard section 30 can be changed by a bending operation of the bendable section 32, and the observation object O can be captured within the observation field of view. Illumination light IL is radiated from the illuminator 20 to the captured observation object O, and the observation object O is illuminated. The bendable section 32 is formed of coupling joint rings (not shown) together in the longitudinal direction of the insertion section 26.

The flexible tube section 34 has desirable flexibility and can be bent when an external force is applied thereto. The flexible tube section 34 is a tubular member extended from a main body 38 (described later) of the handling section 28.

The handling section 28 includes the main body (scope) 38, a grip section 40, and a universal cord 42. The flexible tube section 34 extends from the distal end of the main body 38. The grip section 40 is coupled to the proximal end of the main body 38 and is to be held by the user who operates the endoscope 12. The universal cord connects the grip section 40 and the main body 14 to each other.

In the grip section 40, the bending operation section 36 is arranged so that operation wires (not shown) can be operated to bend the bendable section 32. The bending operation section 36 has a right/left bending operation knob that bends the bendable section 32 rightward or leftward, an up/down bending operation knob 37b that bends the bendable section 32 upward or downward, and a fixing knob 37c that fixes the position of the bent bendable section 32.

A rightward/leftward direction bending operation driving section (not shown), which is driven by an operation of the right/left bending operation knob, is connected to the right/left bending operation knob. An upward/downward direction bending operation driving section (not shown), which is driven by an operation of the up/down bending operation knob, is connected to the up/down bending operation knob. The upward/downward direction bending operation driving section and the rightward/leftward direction bending operation driving section are arranged within, for example, the grip section 40.

The rightward/leftward direction bending operation driving section is connected to a single rightward/leftward direction operation wire (not shown) that is inserted through the handling section 28, flexible tube section 34, and bendable section 32, and both ends of the rightward/leftward direction operation wire are connected to the distal end of the bendable section 32.

The upward/downward direction bending operation driving section is connected to a single upward/downward direction operation wire (not shown) that is inserted through the handling section 28, flexible tube section 34, and bendable section 32. The upward/downward direction operation wire and the rightward/leftward direction operation wire are different members and can be operated independently of each other. Both ends of the upward/downward direction operation wire are connected to the distal end of the bendable section 32.

The right/left bending operation knob bends the bendable section 32 in the rightward/leftward direction through the rightward/leftward direction bending operation driving section and the rightward/leftward operation wire. The up/down bending operation knob bends the bendable section 32 in the upward/downward direction through the upward/downward direction bending operation driving section and the upward/downward direction operation wire.

The bending operation section 36 (right/left bending operation knob and the up/down bending operation knob), the rightward/leftward bending operation driving section, the rightward/leftward direction operation wire, the upward/downward direction bending operation driving section, and the upward/downward direction operation wire jointly constitute a bending operation mechanism that operates the bendable section 32 to bend the bendable section 32.

Each of the structural elements will be described in more detail.

<Input Device 18>

The endoscope apparatus 10 according to the present embodiment has the following four observation modes in accordance with observation purposes, and the user enters which observation mode should be selected for observation by operating the input device 18. Observation mode information entered through the input device 18 is output to the illuminator 20 and the image processor 24.

The observation modes include a superficial blood vessel emphasis mode, an intermediate blood vessel emphasis mode, a deep blood vessel emphasis mode, and a normal observation mode.

The superficial blood vessel emphasis mode is an observation mode in which only blood vessels located in a superficial layer of the observation object O are highlighted.

The intermediate blood vessel emphasis mode is an observation mode in which only blood vessels located in a deep layer of the observation object O are highlighted.

The deep blood vessel emphasis mode is an observation mode in which only blood vessels located in a deep layer of the observation object O are highlighted.

The normal observation mode is an observation mode in which illumination light having high color rendering property or high color reproduction property is radiated. For example, the normal observation mode is an observation mode in which the color of broadband illumination light IL, such as xenon lamp or halogen lamp, is reproduced. Alternatively, the normal observation mode is an observation mode in which the color of observation object O irradiated with broadband illumination light IL, such as xenon lamp or halogen lamp, is reproduced.

<Illuminator 20>

The illuminator 20 includes laser light sources 44-1 to 44-6 (six laser light sources in the present embodiment), a light source driver 46, six optical fibers 48-1 to 48-6, a light combiner 50, an optical fiber 52, and a light converter 54. The laser light sources 44-1 to 44-6, light source driver 46, optical fibers 48-1 to 48-6, light combiner 50, and part of the optical fiber 52 are arranged inside the main body 14, while the remaining port of the optical fiber 52 and the light converter 54 are arranged inside the endoscope 12.

Laser light source 44-1 (laser 1) is a laser light source that has a peak wavelength of 415 nm, and emits first laser light.

Laser light source 44-2 (laser 2) is a laser light source that has a peak wavelength of 445 nm, and emits second laser light.

Laser light source 44-3 (laser 3) is a laser light source that has a peak wavelength of 540 nm, and emits third laser light.

Laser light source 44-4 (laser 4) is a laser light source that has a peak wavelength of 515 nm, and emits fourth laser light.

Laser light source 44-5 (laser 5) is a laser light source that has a peak wavelength of 595 nm, and emits fifth laser light.

Laser light source 44-6 (laser 6) is a laser light source that has a peak wavelength of 635 nm, and emits sixth laser light.

The light source driver 46 controls the driving of these laser light sources 44-1 to 44-6.

The optical fiber 48-1 to 48-6 guide the laser light emitted from the laser light source 44-1 to 44-6 to the light combiner 50.

The light combiner 50 is, for example, an optical fiber combiner, which combines the laser light guided from the laser light sources 44-1 to 44-6 by the optical fibers 48-1 to 48-6.

The optical fiber 52 guides the laser light combined by the light combiner 50 to the light converter 54.

The light converter 54 is disposed in the distal end hard section 30 of the insertion section 26, in which the imager 22 is provided. The light converter 54 converts the optical characteristics of the laser light guided from the main body 14 by the optical fiber 52 inserted through the universal cord 42, handling section 28, and insertion section 26, and radiates the resultant light to the observation object O as illumination light IL.

A more specific description will be given of the structure of each portion of the illuminator 20.

<Laser Light Source 44-1 (Laser 1)>

In the present embodiment, oxyhemoglobin contained in the blood in blood vessels is assumed to be the diagnosis target substance present in the observation object O. FIG. 3 shows an optical absorption spectrum of the oxyhemoglobin (hereinafter referred to simply as hemoglobin).

Laser light source 44-1 (laser 1) is a laser light source that has a peak wavelength of 415 nm. The first laser light whose peak wavelength is 415 nm has a reach length up to the superficial region of the observation object O (the definition of the reach length will be mentioned later). The peak wavelength 415 nm of the first laser light is a maximum wavelength that takes a maximum value in the blue range (the definition of a color range will be mentioned later) of the optical absorption spectrum of the hemoglobin, the diagnosis target substance, and the first laser light is absorbed much in the hemoglobin contained in the blood in the blood vessels in the superficial layer (hereinafter referred to simply as superficial blood vessels). Therefore, where the first laser light is radiated to the observation object O, a large light intensity difference occurs between the light intensity that the reflected and scattered light RL has in the superficial blood vessels and the light intensity that the reflected and scattered light RL has near the superficial blood vessels. In other words, a high contrast is provided for the superficial blood vessels. That is, the superficial blood vessels are emphasized.

Accordingly, the first laser light will be referred to as emphasis narrow band light corresponding to the superficial blood vessels, and laser light source 44-1 (laser 1) will be referred to as an emphasis narrow band light source corresponding to the superficial blood vessels.

The peak wavelength of the first laser light is not limited to 415 nm. The peak wavelength of the first laser light may be another value as long as the peak wavelength or central wavelength is included in the emphasis wavelength range corresponding to the superficial blood vessels.

The emphasis wavelength range corresponding to the superficial blood vessels need not be a wavelength range including a maximum wavelength that takes a maximum value in the blue range of the optical absorption spectrum of the hemoglobin, but may be a wavelength range including a blue-range largest wavelength that takes a largest value in the blue range of the optical absorption spectrum of the hemoglobin.

The emphasis wavelength range corresponding to the superficial blood vessels should preferably be a wavelength range that is within ± 20 nm for at least one of the maximum wavelength that takes a maximum value in the blue range of the optical absorption spectrum of the hemoglobin and the blue-range largest wavelength that takes a largest value in the blue range, because light absorption is great and the superficial blood vessels are emphasized. The emphasis wavelength range should more preferably be within ± 10 nm, because light absorption is greater and the superficial blood vessels are emphasized more.

The emphasis wavelength range corresponding to the superficial blood vessels should preferably be a wavelength range that has values equal to or more than 1/2 of the maximum value in the blue range of the absorption spectrum of the hemoglobin or the largest value in the blue range, because the absorption is great.

In the blue range of the optical absorption spectrum of the hemoglobin, the maximum wavelength and the blue-range largest wavelength are equal to each other.

<Laser Light Source 44-2 (Laser 2)>

Laser light source 44-2 (laser 2) is a laser light source that has a peak wavelength of 445 nm. The second laser light whose peak wavelength is 445 nm has, like the first laser, a reach length up to the superficial region of the observation object O. However, the peak wavelength 445 nm of the second laser light is included in the non-emphasis wavelength range corresponding to the superficial blood vessels, which does not include the above-mentioned emphasis wavelength range corresponding to the superficial blood vessels. Where the second laser light is radiated to the observation object O, a small light intensity difference occurs between the light intensity that the reflected and scattered light RL has in the superficial blood vessels and the light intensity that the reflected and scattered light RL has near the superficial blood vessels. In other words, the second laser light provides a low contrast for the superficial blood vessels. That is, the superficial blood vessels are not emphasized.

Accordingly, the second laser light will be referred to as non-emphasis narrow band light corresponding to the superficial blood vessels, and laser light source 44-2 (laser 2) will be referred to as a non-emphasis narrow band light source corresponding to the superficial blood vessels.

The peak wavelength of the second laser light is not limited to 445 nm. The peak wavelength of the second laser light may be another value as long as it is included in the non-emphasis wavelength range in which the superficial blood vessels are not highlighted.

The non-emphasis wavelength range corresponding to the superficial blood vessels is a range that does not include the emphasis wavelength range corresponding to the superficial blood vessels.

The non-emphasis wavelength range corresponding to the superficial blood vessels is preferably a range that includes at least one of a minimum wavelength that takes a minimum value in the blue range of the optical absorption spectrum of the hemoglobin and a blue-range smallest wavelength that takes a smallest value in the blue range of the optical absorption spectrum of the hemoglobin.

The non-emphasis wavelength range corresponding to the superficial blood vessels should preferably be a wavelength range that is within ± 20 nm for at least one of the above-mentioned minimum wavelength and the smallest wavelength, because light absorption is small and the superficial blood vessels are not emphasized. The non-emphasis wavelength range should more preferably be within ± 10 nm, because light absorption is smaller and the superficial blood vessels are suppressed.

The non-emphasis wavelength range corresponding to the superficial blood vessels should preferably be a wavelength range that has values equal to or less than 1.5 times of at least one of the above-mentioned minimum value and smallest value in the blue range, because light absorption is small.

The non-emphasis wavelength range corresponding to the superficial blood vessels should preferably be a wavelength range that has values equal to or less than 1/2 of at least one of the maximum value in the blue range and the largest value in the blue range, because the absorption is small.

<Laser Light Source 44-3 (Laser 3)>

Laser light source 44-3 (laser 3) is a laser light source that has a peak wavelength of 540 nm. The third laser light whose peak wavelength is 540 nm has a reach length up to the intermediate region of the observation object O, which is deeper than the superficial region. The peak wavelength 540 nm of the third laser light is a maximum wavelength that takes a maximum value in the green range of the optical absorption spectrum of the hemoglobin, and the third laser light is absorbed much in the blood vessels in the intermediate layer. Therefore, where the third laser light is radiated to the observation object O, a large light intensity difference occurs between the light intensity that the reflected and scattered light RL has in the intermediate blood vessels and the light intensity that the reflected and scattered light RL has near the intermediate blood vessels. In other words, a high contrast is provided for the intermediate blood vessels. That is, the intermediate blood vessels are emphasized.

Accordingly, the third laser light will be referred to as emphasis narrow band light corresponding to the intermediate blood vessels, and laser light source 44-3 (laser 3) will be referred to as an emphasis narrow band light source corresponding to the intermediate blood vessels.

The peak wavelength of the third laser light is not limited to 540 nm. The peak wavelength of the third laser light may be another value as long as the peak wavelength or central wavelength is included in the emphasis wavelength range corresponding to the intermediate blood vessels.

The emphasis wavelength range corresponding to the intermediate blood vessels need not be a wavelength range including a maximum wavelength that takes a maximum value in the green range of the optical absorption spectrum of the hemoglobin, but may be a wavelength range including a green-range largest wavelength that takes a largest value in the green range of the optical absorption spectrum of the hemoglobin.

The emphasis wavelength range corresponding to the intermediate blood vessels should preferably be a wavelength range that is within ± 20 nm for at least one of the maximum wavelength that takes a maximum value in the green range of the optical absorption spectrum of the hemoglobin and the green-range largest wavelength that takes a largest value in the green range, because light absorption is great and the intermediate blood vessels are emphasized. The emphasis wavelength range should more preferably be within ± 10 nm, because light absorption is greater and the intermediate blood vessels are emphasized more.

The emphasis wavelength range corresponding to the intermediate blood vessels should preferably be a wavelength range that has values equal to or more than 1/2 of the maximum value in the green range of the absorption spectrum of the hemoglobin or the largest value in the green range, because the absorption is great.

<Laser Light Source 44-4 (Laser 4)>

Laser light source 44-4 (laser 4) is a laser light source that has a peak wavelength of 515 nm. The fourth laser light whose peak wavelength is 515 nm has, like the third laser light, a reach length up to the intermediate region of the observation object O. However, the peak wavelength 515 nm of the fourth laser light is included in the non-emphasis wavelength range corresponding to the intermediate blood vessels, which does not include the above-mentioned emphasis wavelength range corresponding to the intermediate blood vessels. Where the fourth laser light is radiated to the observation object O, a small light intensity difference occurs between the light intensity that the reflected and scattered light RL has in the intermediate blood vessels and the light intensity that the reflected and scattered light RL has near the intermediate blood vessels. In other words, the fourth laser light provides a low contrast for the intermediate blood vessels. That is, the intermediate blood vessels are not emphasized.

Accordingly, the fourth laser light will be referred to as non-emphasis narrow band light corresponding to the intermediate blood vessels, and laser light source 44-4 (laser 4) will be referred to as a non-emphasis narrow band light source corresponding to the intermediate blood vessels.

The peak wavelength of the fourth laser light is not limited to 515 nm. The peak wavelength of the fourth laser light may be another value as long as it is included in the non-emphasis wavelength range in which the intermediate blood vessels are not highlighted.

The non-emphasis wavelength range corresponding to the intermediate blood vessels is a range that does not include the emphasis wavelength range corresponding to the intermediate blood vessels.

The non-emphasis wavelength range corresponding to the intermediate blood vessels is preferably a range that includes at least one of a minimum wavelength that takes a minimum value in the green range of the optical absorption spectrum of the hemoglobin and a green-range smallest wavelength that takes a smallest value in the green range of the optical absorption spectrum of the hemoglobin.

The non-emphasis wavelength range corresponding to the intermediate blood vessels should preferably be a wavelength range that is within $\pm$ 20 nm for at least one of the above-mentioned minimum wavelength and smallest wavelength, because light absorption is small and the intermediate blood vessels are not emphasized. The non-emphasis wavelength range should more preferably be within $\pm$ 10 nm, because light absorption is smaller and the superficial blood vessels are suppressed more.

The non-emphasis wavelength range corresponding to the intermediate blood vessels should preferably be a wavelength range that has values equal to or less than 1.5 times of at least one of the above-mentioned minimum value and smallest value in the green range, because light absorption is small.

Alternatively, the non-emphasis wavelength range corresponding to the intermediate blood vessels should preferably be a wavelength range that has values equal to or less than 1/2 of at least one of the maximum value in the green range and the largest value in the green range, because the absorption is small.

<Laser Light Source 44-5 (Laser 5)>

Laser light source 44-5 (laser 5) is a laser light source that has a peak wavelength of 595 nm. The fifth laser light whose peak wavelength is 595 nm has a reach length up to a deep region of the observation object O, which is deeper than the intermediate region. The peak wavelength 595 nm of the fifth laser light is included in the emphasis wavelength range corresponding to the deep blood vessels, i.e., a wavelength range that is within $\pm$ 20 nm for the red-range largest wavelength 590 nm that takes a largest value in the red range of the optical absorption spectrum of the hemoglobin, and that has values equal to or more than 1/2 of the red-range largest value, and absorption in the deep blood vessels is great. Therefore, where the fifth laser light is radiated to the observation object O, a large light intensity difference occurs between the light intensity that the reflected and scattered light RL has in the deep blood vessels and the light intensity that the reflected and scattered light RL has near the deep blood vessels. In other words, a high contrast is provided for the deep blood vessels. That is, the deep blood vessels are emphasized.

Accordingly, the fifth laser light will be referred to as emphasis narrow band light corresponding to the deep blood vessels, and laser light source 44-5 (laser 5) will be referred to as an emphasis narrow band light source corresponding to the deep blood vessels.

The peak wavelength of the fifth laser light is not limited to 595 nm. The peak wavelength of the fifth laser light may be another value as long as the peak wavelength or central wavelength is included in the emphasis wavelength range corresponding to the deep blood vessels.

The emphasis wavelength range corresponding to the deep blood vessels need not be a wavelength range including a maximum wavelength that takes a maximum value in the red range of the optical absorption spectrum of the hemoglobin, but may be a wavelength range including a red-range largest wavelength that takes a largest value in the red range of the optical absorption spectrum of the hemoglobin.

The emphasis wavelength range corresponding to the deep blood vessels should preferably be a wavelength range that is within $\pm$ 20 nm for at least one of the maximum wavelength that takes a maximum value in the red range of the optical absorption spectrum of the hemoglobin and the red-range largest wavelength that takes a largest value in the red range, because light absorption is great and the deep blood vessels are emphasized. The emphasis wavelength range should more preferably be within $\pm$ 10 nm, because light absorption is greater and the deep blood vessels are emphasized more.

The emphasis wavelength range corresponding to the deep blood vessels should preferably be a wavelength range that has values equal to or more than 1/2 of the maximum value in the red range of the absorption spectrum of the hemoglobin or the largest value in the red range, because the absorption is great.

<Laser Light Source 44-6 (Laser 6)>

Laser light source 44-6 (laser 6) is a laser light source that has a peak wavelength of 635 nm. The sixth laser light whose peak wavelength is 635 nm has, like the fifth laser light, a reach length up to the deep region of the observation object O. However, the peak wavelength 635 nm of the sixth laser light is included in the non-emphasis wavelength range corresponding to the deep blood vessels, which does not include the above-mentioned emphasis wavelength range corresponding to the deep blood vessels. Where the sixth laser light is radiated to the observation object O, a small light intensity difference occurs between the light intensity that the reflected and scattered light RL has in the deep blood vessels and the light intensity that the reflected and scattered light RL has near the deep blood vessels. In other words, a low contrast is provided for the deep blood vessels. That is, the deep blood vessels are not emphasized.

Accordingly, the sixth laser light will be referred to as non-emphasis narrow band light corresponding to the deep blood vessels, and laser light source 44-6 (laser 6) will be referred to as a non-emphasis narrow band light source corresponding to the deep blood vessels.

The peak wavelength of the sixth laser light is not limited to 635 nm. The peak wavelength of the sixth laser light may be another value as long as it is included in the non-emphasis wavelength range in which the deep blood vessels are not highlighted.

The non-emphasis wavelength range corresponding to the deep blood vessels is a range that does not include the emphasis wavelength range corresponding to the deep blood vessels.

The non-emphasis wavelength range corresponding to the deep blood vessels is preferably a range that includes at least one of a minimum wavelength that takes a minimum value in the red range of the optical absorption spectrum of the hemoglobin and a red-range smallest wavelength that takes a smallest value in the red range of the optical absorption spectrum of the hemoglobin.

The non-emphasis wavelength range corresponding to the deep blood vessels should preferably be a wavelength range that is within ± 20 nm for at least one of the above-mentioned minimum wavelength and smallest wavelength, because light absorption is small and the deep blood vessels are not emphasized. The non-emphasis wavelength range should more preferably be within ± 10 nm, because light absorption is smaller and the deep blood vessels are suppressed more.

The non-emphasis wavelength range corresponding to the deep blood vessels should preferably be a wavelength range than has values equal to or less than 1.5 times of at least one the above-mentioned minimum value and smallest value in the red range, because light absorption is small.

Alternatively, the non-emphasis wavelength range corresponding to the deep blood vessels should preferably be a wavelength range that has values equal to or less than 1/2 of at least one of the maximum value in the red range and the largest value in the red range, because the absorption is small.

It should be noted the narrow band light mentioned above, i.e., the emphasis narrow band light and the non-emphasis narrow band light, may be light other than laser light. The narrow band light should preferably be light having a wavelength width of 50 nm or less, more preferably light having a wavelength width of 5 nm or less. The wavelength width is, for example, a wavelength width defined by the full width at half maximum (FWHM) or the root mean square (RMS). The wavelength width of half-value-width laser light is, for example, 1 nm. A light source may be, for example, an LED or a light source using fluorescent light exited by LED light or laser light; alternatively, the light source may generate narrow band light from broadband light using spectral filters. In a structure that uses the spectral filters to generate narrow band light, wavelengths of radiated narrow band light are switched from one to another by mechanically switching the spectral filters.

<Color Ranges>

The blue range, green range, and red range mentioned above are defined by the following wavelength ranges:

Blue Range: 400 to 510 nm
Green Range: 490 to 610 nm
Red Range: 590 to 700 nm

These wavelength ranges are wavelength ranges obtained by dividing a wavelength range from 400 to 700 nm of the visible light range equally into three ranges and providing an overlap of 20 nm between the adjacent ranges. Where wavelengths are set based on these well-balanced wavelength ranges, and light have wavelengths that are within the respective color ranges of the blue range, green range, and red range, illumination light IL having good color reproduction property can be generated.

For example, a wavelength range that is less than 400 nm and a wavelength range that is 700 nm or more may be allocated to the blue range and the red range, respectively. In this case, the blue range, green range, and red range are defined by the following wavelength ranges:

Blue Range: 380 to 510 nm
Green Range: 490 to 610 nm
Red Range: 590 to 780 nm

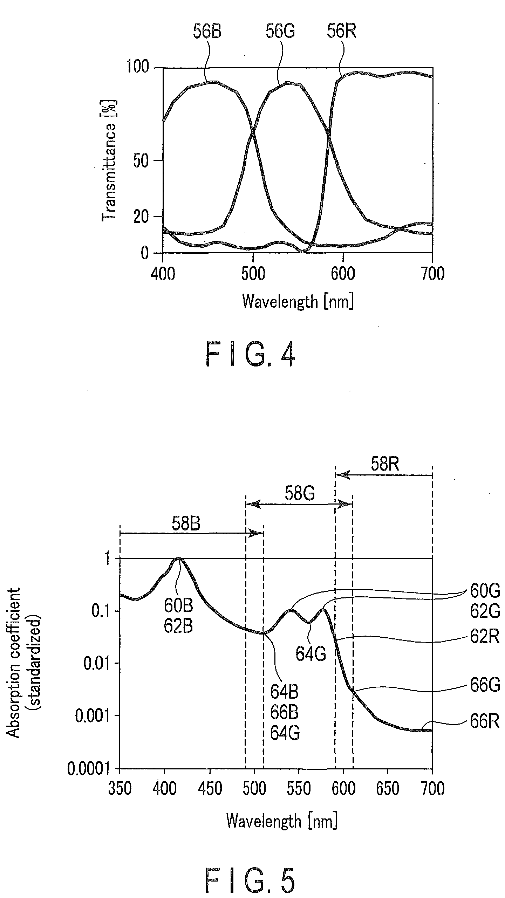

For example, when the imager 22 acquires a spectral image, using the color filters, the blue range, green range, and red range may be defined using the spectroscopic characteristics of the color filters. FIG. 4 shows an example of the spectroscopic characteristics 56B of the blue (B) color filter, the spectroscopic characteristics 56G of the green (G) color filter, and the spectroscopic characteristics 56R of the red (B) color filter. Let us assume that a wavelength range having a transmittance of 20% or more is defined as the color range of each color filter. As shown in FIG. 4, the blue range is 400 to 525 nm, the green range is 470 to 625 nm, and the red range is 570 to 700 nm.

As shown in FIG. 4, there is hardly any wavelength range in which the transmittance of the color filters is zero, and the transmittance is several % to 10% or so in a broad range of the visible light. The transmittance of several % to 10% or so can be regarded as a negligible level in the photographing a color image, so that color ranges should be preferably defined based on the range in which the transmittance is 20% or higher.

<Maximum Value and Color-Range Largest Value in Each Color Range>

How a maximum value and a color-range largest value for the absorption spectrum of oxyhemoglobin are in each color range is shown in FIG. 5.

In the blue range 58B, the maximum wavelength that takes the blue-range maximum value 60B and the color-range largest wavelength that takes the blue-range largest value 62B are the same wavelength 415 nm, and the minimum wavelength that takes the blue-range minimum value 64B and the color-range minimum wavelength that takes the blue-range smallest value 66B takes are the same wavelength 500 nm.

In contrast, in the green range 58G, the maximum wavelength that takes the green-range maximum value 60G and the color-range largest wavelength that takes the green-range largest value 62G are the same wavelength, but this wavelength appears at two points, i.e., at 540 nm and approximately 575 nm. The minimum wavelength that takes the green-range minimum value 64G also appears at two points, i.e., at 500 nm and 560 nm. The color-range minimum wavelength that takes the green-range smallest value 66G is wavelength 610 nm.

In the red range 58R, neither a maximum value nor a minimum value exists, the color-range largest wavelength that takes the red-range largest value 62R is wavelength 590 nm, and the color-range smallest wavelength that takes the red-range smallest value 66R is wavelength 685 nm.

<Reach Length>

Where light of a wavelength range from near ultraviolet to near infrared is radiated to a living body (observation object O), light of a longer wavelength travels deeper into the living body, due to the light scattering property and light absorption property in living tissues (an epithelial tissue, a mucous membrane, a body fluid, etc.)

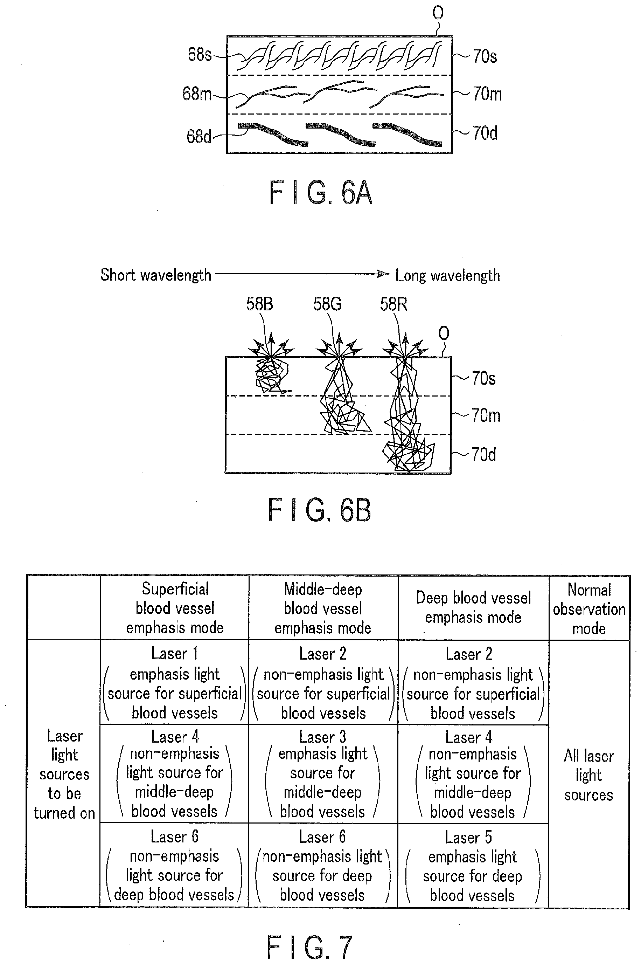

For example, as shown in FIG. 6A, the blood vessels of a living body (observation object O) include superficial blood vessels (capillaries) 68s located near the surface of the living body, intermediate blood vessels (blood vessels thicker than the capillaries) 68m located in deeper portions, and deep blood vessels (blood vessels thicker than the intermediate blood vessels) 68d located in further deeper portions. The region where the superficial blood vessels 68s exist will be referred to as a superficial region 70s of the living body, the region where the intermediate blood vessels 68m exist will be referred to as an intermediate region 70m, and the region where the deep blood vessels 68d exist will be referred to as a deep region 70d.

As shown in FIG. 6B, where light of the blue range 58B on the short wavelength side is radiated to the living body (observation objection O), the light of the blue range 58B has a reach length up to the superficial region 70s of the living body, is greatly influenced by the absorption by the superficial blood vessels 68s, and the results are reflected in an image of the living body (observation object O). Where light of the green range 58G is radiated, the light of the green range 58G has a reach length up to the intermediate region 70m of the living body, is greatly influenced by the absorption by the intermediate blood vessels 68m, and the results are reflected in an image of the living body (observation object O). Where light of the red range 58R is radiated, the light of the red range 58R has a reach length up to the deep region 70d of the living body, is greatly influenced by the absorption by the deep blood vessels 68d, and the results are reflected in an image of the living body (observation object O).

For example, the reach length is defined as follows:
Light intensity $I(x)$ at distance x within a living body (observation object O) is expressed by $I(x) = I_0 \exp[-\alpha x]$, where $I_0$ is an incident light intensity and $\alpha$ is an attenuation coefficient.

The reach length is defined as the reciprocal of attenuation coefficient $\alpha$, i.e., a distance at which the light intensity become equal to $1/e$. Attenuation coefficient $\alpha$ is defined by equation (1) set forth below, provided that $\mu_a$ is an absorption coefficient, $\mu_s$ is a scattering coefficient, g is an anisotropy factor, and an equivalent scattering coefficient is given by $\mu_s' = (1 - g)\mu_s$.

$$\alpha = \sqrt{(3\mu_a(\mu_a + \mu_s'))} \qquad \cdots (1)$$

For example, absorption coefficient $\mu_a$, scattering coefficient $\mu_s$, and equivalent scattering coefficient $\mu_s$' may be merely used as attenuation coefficient $\alpha$.

Absorption coefficient $\mu_a$, scattering coefficient $\mu_s$, and anisotropy factor g are determined depending upon the living body (observation object O) and the wavelength.

<Optical Fibers 48-1 to 48-6 and 52>

The optical fibers 48-1 to 48-6 and the optical fiber 52 are single-wire fibers having a core diameter of, for example, several tens of $\mu$m to several hundreds of $\mu$m. An optical coupling lens (not shown in the drawings) is disposed between each of the laser light sources 44-1 to 44-6 and the optical fibers 48-1 to 48-6 to converge the laser light emitted from the laser sources and couple it to the optical fibers.

In place of the optical fiber 52, a bundle fiber made of a bundle of optical fibers may be used.

<Light Source Driver 46>

The light source driver 46 is capable of controlling the ON/OFF, driving currents, and driving systems (continuous driving (CW), pulse driving, etc.) of the laser light sources, for each of the laser light sources independently.

The light source driver 46 controls how the laser light sources 44-1 to 44-6 should be turned on in combination in accordance with observation mode information supplied from the input device 18.

The light source driver 46 includes a storage 72 that has stored combinations of laser light sources to be turned on in each observation mode and the current value or voltage value to be applied to each laser light source.

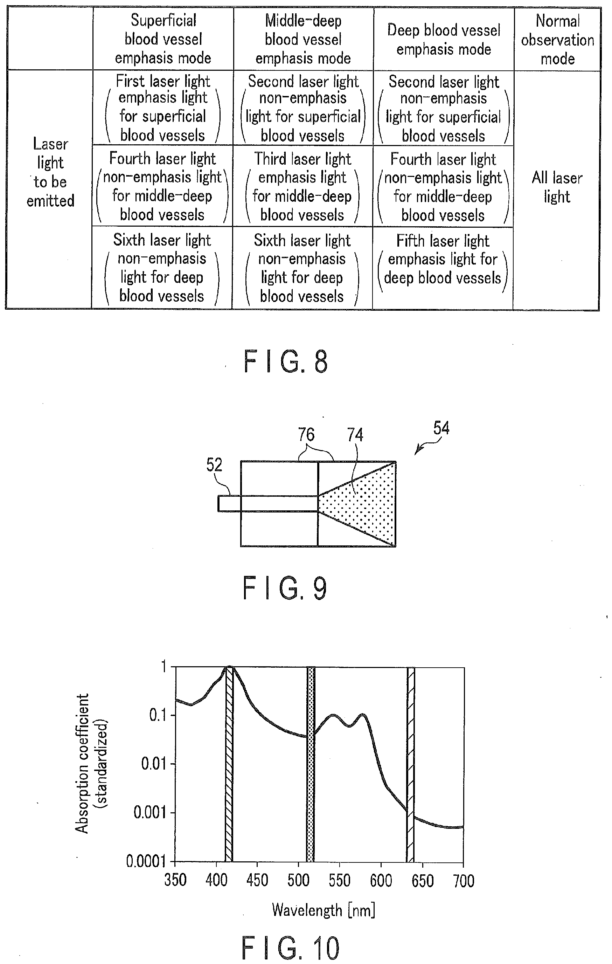

That is, the storage 72 has stored combinations of laser light sources to be turned on in each of the observation modes shown in FIG. 7 and combinations of laser light to be radiated in each of the observation modes shown in FIG. 8.

The light source driver 46 may be constituted by a processor. In this case, the storage 72 may be a built-in storage of the processor, or may be an external storage accessible by the processor. The external storage has stored a program code that causes the processor to function as the light source driver 46 when executed by the processor.

Details of the combinations of laser light sources to be turned on and the combinations of rays of laser light to be radiated in each observation mode will be mentioned later.

<Light Converter 54>

As shown in FIG. 9, the light converter 54 includes a diffusing member 74 located at the distal end of the optical fiber 52 and formed of alumina particles or the like. The distal end of the optical fiber 52 and the diffusing member 74 are held by a holder 76, and the positional relation between them is defined.

The diffusing member 74 has a function of diffusing laser light guided by the optical fiber 52 and changing it to light having a desirable light distribution. The diffusing member 74 does not convert the wavelengths of the light.

In place of the diffusing member 74, the light converter 54 may employ a lens or a combination of the lens and the diffusing member 74.

Where a bundle fiber is employed in place of the optical fiber 52, the light converter 54 may employ a lens in place of the diffusing member 74.

<Imager 22>

The imager 22 detects reflected and scattered light RL from the observation object O to generate an imaging signal. The imaging signal is output to the image processor 24 of the main body 14.

Although not shown in the drawings, the imager 22 includes three type of light detection elements, which are an R light detection element to detect the red range 58R, a G light detection element to detect the green range 58G, and a B light detection element to detect the blue range 58B. Examples of the spectroscopic characteristics of the color filters of the R light detection element, G light detection element, and B light detection element are shown in FIG. 4.

The imager 22 uses the R light detection element, G light detection element, and B light detection element to generate an R imaging signal, a G imaging signal, and a B imaging signal for the red range 58R, the green range 58G, and the blue range 58B, separately and independently.

The imager 22 is specifically a CCD imager or a CMOS imager.

The imager 22 may be a monochromatic imager having no color filter. In this case, the imager sequentially receives reflected and scattered light RL of laser light sequentially emitted at different timings, so as to generate imaging signals therefrom, and the image processor 24 performs RGB assignment processing. <Image Processor 24 and Image Display 16>

The image processor 24 performs image processing for the B imaging signal, G imaging signal, and R imaging signal output from the imager 22 in accordance with observation mode information, so as to generate an image signal constituting an observation object image.

The image processor 24 may be constituted by a processor. In this case, an external storage accessible by the processor has stored a program code that causes the processor to function as the image processor 24 when executed by the processor.

Where the imager 22 is a monochromatic imager with no color filter, RGB assignment processing is first performed for imaging signals sequentially generated at different timings, and then an image signal is generated.

The image display 16 displays an observation object image in accordance with an image signal generated by the image processor 24. The image display 16 is, for example, a monitor such as a liquid crystal display.

An operation of the endoscope apparatus 10 having the above structure will be described.

A detailed description will be given of the case where the superficial blood vessel emphasis mode is entered from the input device 18 by the user, the case where the intermediate blood vessel emphasis mode is entered, and the case where the deep blood vessel emphasis mode is entered.

<Superficial Blood Vessel Emphasis Mode>

Where the user enters the superficial blood vessel emphasis mode from the input device 18 as an observation mode, the input device 18 outputs observation mode information on the superficial blood vessel emphasis mode to the light source driver 46 and the image processor 24.

Upon receipt of the observation mode information on the superficial blood vessel emphasis mode, the light source driver 46 turns on laser light source 44-1 (laser 1), laser light source 44-4 (laser 4), and laser light source 44-6 (laser 6), so as to cause the laser light sources 44-1, 44-4, and 44-6 to emit first laser light, fourth laser light, and sixth laser light.

Laser light source 44-1 (laser 1) is an emphasis narrow band light source corresponding to the superficial blood vessels 68s (superficial region 70s), and the first laser light emitted from laser light source 44-1 (laser 1) is emphasis narrow band light corresponding to the superficial blood vessels 68s (superficial region 70s). The wavelength of the first laser light, the emphasis narrow band light corresponding to the superficial blood vessels 68s, is 415 nm and is included in the blue range 58B, as shown in FIG. 10. In FIG. 10, the ordinate axis of the laser light spectrum is drawn in an arbitrary scale.

Laser light source 44-4 (laser 4) is a non-emphasis narrow band light source corresponding to the intermediate blood vessels 68m (intermediate region 70m), and the fourth laser light emitted from laser light source 44-4 (laser 4) is non-emphasis narrow band light corresponding to the intermediate blood vessels 68m (intermediate region 70m). The wavelength of the fourth laser light, the non-emphasis narrow band light corresponding to the intermediate blood vessels 68m, is 515 nm and is included in the green range 58G, as shown in FIG. 10.

Laser light source 44-6 (laser 6) is a non-emphasis narrow band light source corresponding to the deep blood vessels 68d (deep region 70d), and the sixth laser light emitted from laser light source 44-6 (laser 6) is non-emphasis narrow band light corresponding to the deep blood vessels 68d (deep region 70d). The wavelength of the sixth laser light, the non-emphasis narrow band light corresponding to the deep blood vessels 68d, is 635 nm and is included in the red range 58R, as shown in FIG. 10.

After being guided by the optical fibers 48-1, 48-4, and 48-6, rays of the first laser light, fourth laser light, and sixth laser light are combined together by the light combiner 50.

The combined ray of the first laser light, fourth laser light, and sixth laser light is converted into light having a desirable light distribution by the light converter 54 at the distal end of the insertion section 26, and the resultant light is radiated to the observation object O as illumination light IL.

The first laser light whose wavelength is included in the blue range 58B has a reach length up to the superficial region 70s. Where the first laser light is radiated to the observation object O, a large light intensity difference occurs between the light intensity that the reflected and scattered light RL has in the superficial blood vessels and the light intensity that the reflected and scattered light RL has near the superficial blood vessels. In other words, a high contrast is provided for the superficial blood vessels 68s. That is, the superficial blood vessels are emphasized.

The reflected and scattered light RL of the illumination light IL in the observation object O is detected by the imager 22. The imager 22 detects the reflected and scattered light RL of the first laser light whose wavelength is included in the blue range 58B with the B light detection element, so as to generate a B imaging signal. The B imaging signal is output to the image processor 24. The image processor 24 performs image processing for the B imaging signal output from the imager 22 in accordance with observation mode information, so as to generate a B image signal. An image signal generated from the imaging signal acquired with emphasis narrow band light will be referred to as an emphasis image signal. In the superficial blood vessel emphasis mode, therefore, the B image signal generated from the B imaging signal acquired with the first laser light, emphasis narrow band light, is an emphasis image signal.

The fourth laser light whose wavelength is included in the green range 58G has a reach length up to the intermediate region 70m. Where the fourth laser light is radiated to the observation object O, a small light intensity difference occurs between the light intensity that the reflected and scattered light RL has in the intermediate blood vessels 68m and the light intensity that the reflected and scattered light RL has near the intermediate blood vessels 68m. In other words, a low contrast is provided for the intermediate blood vessels 68m. That is, the intermediate blood vessels 68m are not emphasized.

The imager 22 detects the reflected and scattered light RL of the fourth laser light whose wavelength is included in the green range 58G with the G light detection element, so as to generate a G imaging signal. The G imaging signal is output to the image processor 24. The image processor 24 performs image processing for the G imaging signal output from the imager 22 in accordance with observation mode information, so as to generate a G image signal. An image signal generated from the imaging signal acquired with non-emphasis narrow band light will be referred to as a non-emphasis image signal. In the superficial blood vessel emphasis mode, therefore, the G image signal generated from the G imaging signal acquired with the fourth laser light, non-emphasis narrow band light, is a non-emphasis image signal.

The sixth laser light whose wavelength is included in the red range 58R has a reach length up to the deep region 70d. Where the sixth laser light is radiated to the observation object O, a small light intensity difference occurs between the light intensity that the reflected and scattered light RL has in the deep blood vessels 68d and the light intensity that the reflected and scattered light RL has near the deep blood vessels 68d. In other words, a low contrast is provided for the deep blood vessels 68d. That is, the deep blood vessels 68d are not emphasized.

The imager 22 detects the reflected and scattered light RL of the sixth laser light whose wavelength is included in the red range 58R with the R light detection element, so as to generate an R imaging signal. The R imaging signal is output to the image processor 24. The image processor 24 performs image processing for the R imaging signal output from the imager 22 in accordance with observation mode information, so as to generate an R image signal. In the superficial blood vessel emphasis mode, the R image signal generated from the R imaging signal acquired with the sixth laser light, non-emphasis narrow band light, is a non-emphasis image signal.

That is, the light intensity that the reflected and scattered light RL of the illumination light IL has in the superficial blood vessels 68s has a larger intensity difference with respect to the light intensity that the reflected and scattered light RL has near the blood vessels (in the mucous membrane or the like) than that of the light intensity that the reflected and scattered light RL has in the intermediate blood vessels 68m and the deep blood vessels 68d.

In the illumination light IL in the superficial blood vessel emphasis mode, the superficial region 70s is an attention depth region, and the intermediate region 70m and the deep region 70d are non-attention depth regions.

This observation mode is effective in observing the superficial blood vessels 68s in detail.

In the illumination light IL, emphasis narrow band light is included in any one color range of the three color ranges. Owing to this, only the superficial layer, or as will be described below, either the intermediate layer or deep layer, can be emphasized. It should be noted that "any one color range" does not exclude a color overlap range that overlaps another color range. The color range is a single-color range including the color overlap range.

The illumination light IL includes single emphasis narrow band light. Non-emphasis narrow band light of the illumination light IL is not included in the color range that includes the emphasis narrow band light. Owing to this, the blood vessels located in an attention depth region can be emphasized. This is because, if different emphasis narrow band light are included in the attention depth region or if emphasis narrow band light is mixed with non-emphasis narrow band light, the blood vessel contrast in the attention depth may be decreased.

The first laser light (emphasis narrow band light), the fourth laser light (non-emphasis narrow band light), and the sixth laser light (non-emphasis narrow band light) may be simultaneously turned on to irradiate the observation object O; alternatively, they may be turned on sequentially at different timings. In particular, where the imager 22 is a monochromatic imager with no color filter, they have to be sequentially turned on and radiated at different timings.

Emphasis narrow band light and non-emphasis narrow band light (the fourth laser light in this case) included in a color range (the green range 58G in this case) adjacent to the color range including the emphasis narrow band light should preferably be emitted sequentially at different timings, and the imager 22 should preferably separate each light into a B imaging signal and a G imaging signal. In many cases, the color filters of the imager 22 have sensitivity to adjacent color ranges. In this case, an imaging signal (the B imaging signal in this case) including emphasis narrow band light may also include non-emphasis narrow band light (the fourth laser in this case), lowering the blood vessel contrast in the attention depth.

The intensity ratio among the first laser light, fourth laser light, and sixth laser light is determined so that the mixed light of the first, fourth, and sixth laser light is white light. White light is light in which the color of broadband illumination light IL, such as xenon lamp or halogen lamp, is reproduced. Alternatively, white light is light in which the color of observation object O irradiated with broadband illumination light IL, such as xenon lamp or halogen lamp, is reproduced. More specifically, white light is defined using, for example, chromaticity coordinates, a correlated color temperature, or a color difference from a black body locus. For example, it is defined as, in the chromaticity coordinates, a color within the ranges ($x = 0.2$-$0.4$, $y = 0.2$-$0.4$) and ($x = 0.4$-$0.5$, $y = 0.35$-$0.45$), in the correlated color temperature, a color of the range from 2000 to 100000K, or in the black body locus, a color of the range in which the color difference (duv) from the black body locus is ± 0.1 or less. White light may be defined in consideration of the spectral sensitivity of an imaging element. For example, white light may be defined as above, using the chromaticity coordinates or correlated color temperature calculated for the spectrum obtained by multiplying the spectrum of illumination light IL with the spectral sensitivity of the imaging element.

It may be set to have a color other than the white color in accordance with the purpose of use. In this case as well, the color is defined using the chromaticity coordinates or the like.

The two rays of non-emphasis narrow band light of the illumination light IL are respectively included in the two color ranges (the green range 58G and red range 58R in this case) that do not include the emphasis narrow band light. The two rays of non-emphasis narrow band light are included in the narrow band ranges constituting the illumination light IL so as to enhance the color reproduction property of the illumination light IL. In order to enhance the color reproduction property of the illumination light IL, it is desirable that non-emphasis narrow band light is included in, of the three color ranges, all color ranges that do not include the emphasis narrow band light, but it is only required that at least a ray of non-emphasis narrow band light is included in a color range that does not include the emphasis narrow band light (for example, only the first laser light and fourth laser light, or only the first laser and sixth laser light). Owing to this, the color reproduction property of the illumination light IL is enhanced. Further, either the emphasis narrow band light or the non-emphasis narrow band light should preferably be included in each of the three color ranges, in order to enhance the color reproduction property.

The image processor 24 performs at least one of the contrast emphasis image process, outline (edge) emphasis image process, and blood vessel structure image process for the imaging signal that is one of the B imaging signal, G imaging signal, and R imaging signal and that corresponds to the color range including the emphasis narrow band light.

The three image processes are known image processes per se.

The contrast emphasis image process is an image process in which the image brightness difference (contrast) is increased.

The outline (edge) emphasis image process is an image process in which the brightness difference at an outline (edge) portion (a brightness changing portion) in an image is increased.

The blood vessel structure image process is an image process in which the frequency components corresponding to the blood vessel patterns are emphasized.

The image processor 24 performs at least one of the contrast suppression image process, outline (edge) suppression image process, and blood vessel structure suppression image process for the imaging signal that is one of the B imaging signal, G imaging signal, and R imaging signal and that corresponds to the color range not including the emphasis narrow band light.

The three image processes are known processes per se.

That is, the contrast emphasis image process is a process in which the image brightness difference (contrast) is decreased.

The outline (edge) emphasis image process is an image process in which the brightness difference at an outline (an edge or a brightness changing portion) in an image is decreased.

The blood vessel structure image process is an image process in which the frequency components corresponding to the blood vessel patterns are suppressed.

Where the emphasis narrow band light and the non-emphasis narrow band light are included only in two color ranges (for example, the case where only the first laser light and the fourth laser light are used), an observation object image may be generated by assigning two imaging signals to three image signals in a known color conversion process (for example, an R image is generated from a G imaging signal, and G and B images are generated from a B imaging signal).

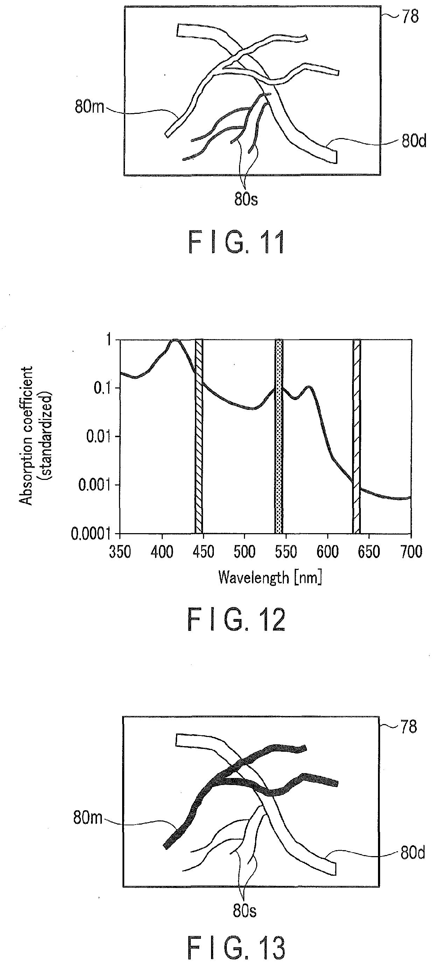

The emphasis image signal and the non-emphasis image signal generated by the image processor 24 are transmitted to the image display 16 to be displayed as an observation object image 78, as shown in FIG. 11. That is, in this observation object image 78, the superficial blood vessel image 80s showing the superficial blood vessels 68s is highlighted, while the intermediate blood vessel image 80m and the deep blood vessel image 80d respectively showing the intermediate blood vessels 68m and deep blood vessels 68d are not highlighted.

<Intermediate Blood Vessel Emphasis Mode>

Where the user enters the intermediate blood vessel emphasis mode from the input device 18 as an observation mode, the input device 18 outputs observation mode information on the intermediate blood vessel emphasis mode to the light source driver 46 and the image processor 24.

Upon receipt of the observation mode information on the intermediate blood vessel emphasis mode, the light source driver 46 turns on laser light source 44-2 (laser 2), laser light source 44-3 (laser 3), and laser light source 44-6 (laser 6), so as to cause the laser light sources 44-2, 44-3, and 44-6 to emit second laser light, third laser light, and sixth laser light.

Laser light source 44-2 (laser 2) is a non-emphasis narrow band light source corresponding to the superficial blood vessels 68s (superficial region 70s), and the second laser light emitted from laser light source 44-2 (laser 2) is non-emphasis narrow band light corresponding to the superficial blood vessels 68s (superficial region 70s). The wavelength of the second laser light, the non-emphasis narrow band light corresponding to the superficial blood vessels 68s, is 445 nm and is included in the blue range 58B, as shown in FIG. 12. In FIG. 12, the ordinate axis of the laser light spectrum is drawn in an arbitrary scale.

Laser light source 44-3 (laser 3) is an emphasis narrow band light source corresponding to the intermediate blood vessels 68m (intermediate region 70m), and the third laser light emitted from laser light source 44-3 (laser 3) is emphasis narrow band light corresponding to the intermediate blood vessels 68m (intermediate region 70m). The wavelength of the third laser light, the emphasis narrow band light corresponding to the intermediate blood vessels 68m, is 540 nm and is included in the green range 58G, as shown in FIG. 12.

Laser light source 44-6 (laser 6) is a non-emphasis narrow band light source corresponding to the deep blood vessels 68d (deep region 70d), and the sixth laser light emitted from laser light source 44-6 (laser 6) is non-emphasis narrow band light corresponding to the deep blood vessels 68d (deep region 70d). The wavelength of the sixth laser light, the non-emphasis narrow band light corresponding to the deep blood vessels 68d, is 635 nm and is included in the red range 58R, as shown in FIG. 12.

After being guided by the optical fibers 48-2, 48-3, and 48-6, rays of the second laser light, third laser light, and sixth laser light are combined together by the light combiner 50.

The combined ray of the second laser light, third laser light, and sixth laser light is converted into light having a desirable light distribution by the light converter 54 at the distal end of the insertion section 26, and the resultant light is radiated to the observation object O as illumination light IL.

The second laser light whose wavelength is included in the blue range 58B has a reach length up to the superficial region 70s. Where the second laser light is radiated to the observation object O, a small light intensity difference occurs between the light intensity that the reflected and scattered light RL has in the superficial blood vessels 68s and the light intensity that the reflected and scattered light RL has near the superficial blood vessels 68s. In other words, a low contrast is provided for the superficial blood vessels 68s. That is, the superficial blood vessels 68s are not emphasized.

The reflected and scattered light RL of the illumination light IL in the observation object O is detected by the imager 22. The imager 22 detects the reflected and scattered light RL of the second laser light whose wavelength is included in the blue range 58B with the B light detection element, so as to generate a B imaging signal. The B imaging signal is output to the image processor 24. The image processor 24 performs image processing for the B imaging signal output from the imager 22 in accordance with observation mode information, so as to generate a B image signal. In the intermediate blood vessel emphasis mode, the B image signal generated from the B imaging signal acquired with the second laser light, non-emphasis narrow band light, is a non-emphasis image signal.

The third laser light whose wavelength is included in the green range 58G has a reach length up to the intermediate region 70m. Where the third laser light is radiated to the observation object O, a large light intensity difference occurs between the light intensity that the reflected and scattered light RL has in the intermediate blood vessels 68m and the light intensity that the reflected and scattered light RL has near the intermediate blood vessels 68m. In other words, a high contrast is provided for the intermediate blood vessels 68m. That is, the intermediate blood vessels 68m are emphasized.

The imager 22 detects the reflected and scattered light RL of the third laser light whose wavelength is included in the green range 58G with the G light detection element, so as to generate a G imaging signal. The G imaging signal is output to the image processor 24. The image processor 24 performs image processing for the G imaging signal output from the imager 22 in accordance with observation mode information, so as to generate a G image signal. In the intermediate blood vessel emphasis mode, the G image signal generated from the G imaging signal acquired with the third laser light, emphasis narrow band light, is an emphasis image signal.

The sixth laser light whose wavelength is included in the red range 58R has a reach length up to the deep region 70d. Where the sixth laser light is radiated to the observation object O, a small light intensity difference occurs between the light intensity that the reflected and scattered light RL has in the deep blood vessels 68d and the light intensity that the reflected and scattered light RL has near the deep blood vessels 68d. In other words, a low contrast is provided for the deep blood vessels 68d. That is, the deep blood vessels 68d are not emphasized.

The imager 22 detects the reflected and scattered light RL of the sixth laser light whose wavelength is included in the red range 58R with the R light detection element, so as to generate an R imaging signal. The R imaging signal is output to the image processor 24. The image processor 24 performs image processing for the R imaging signal output from the imager 22 in accordance with observation mode information, so as to generate an R image signal. In the intermediate blood vessel emphasis mode, the R image signal generated from the R imaging signal acquired with the sixth laser light, non-emphasis narrow band light, is a non-emphasis image signal.

That is, the light intensity that the reflected and scattered light RL of the illumination light IL has in the intermediate blood vessels 68m has a larger intensity difference with respect to the light intensity that the reflected and scattered light RL has near the blood vessels (in the mucous membrane or the like) than that of the light intensity that the reflected and scattered light RL has in the superficial blood vessels 68s and the deep blood vessels 68d.

In the illumination light IL in the intermediate blood vessel emphasis mode, the intermediate region 70m is an attention depth region, and the superficial region 70s and the deep region 70d are non-attention depth regions.

This observation mode is effective in observing the intermediate blood vessels 68m in detail.

The image processor 24 performs at least one of the contrast emphasis image process, outline (edge) emphasis image process, and blood vessel structure image process for the imaging signal (the G imaging signal in this case) that is one of the B imaging signal, G imaging signal, and R imaging signal and that corresponds to the color range including the emphasis narrow band light. The image processor 24 performs at least one of the contrast suppression image process, outline (edge) suppression image process, and blood vessel structure suppression image process for the imaging signals (the B imaging signal and the R imaging signal in this case) that are part of the B imaging signal, G imaging signal, and R imaging signal and that correspond to the color range not including the emphasis narrow band light.

The emphasis image signal and the non-emphasis image signal generated by the image processor 24 are transmitted to the image display 16 to be displayed as an observation object image 78, as shown in FIG. 13. That is, in this observation object image 78, the intermediate blood vessel image 80m showing the intermediate blood vessels 68m is highlighted, while the superficial blood vessel image 80s and the deep blood vessel image 80d showing the superficial blood vessels 68s and deep blood vessels 68d are not highlighted.

<Deep Blood Vessel Emphasis Mode>

Where the user enters the deep blood vessel emphasis mode from the input device 18 as an observation mode, the input device 18 outputs observation mode information on the deep blood vessel emphasis mode to the light source driver 46 and the image processor 24.

Upon receipt of the observation mode information on the deep blood vessel emphasis mode, the light source driver 46 turns on laser light source 44-2 (laser 2), laser light source 44-4 (laser 4), and laser light source 44-5 (laser 5), so as to cause the laser light sources 44-2, 44-4, and 44-5 to emit second laser light, fourth laser light, and fifth laser light.

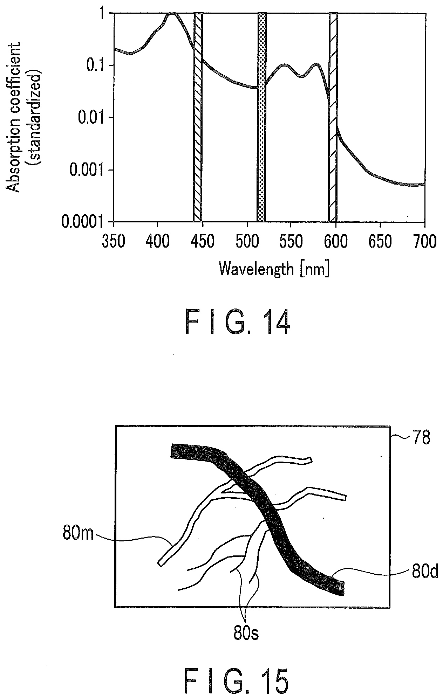

Laser light source 44-2 (laser 2) is a non-emphasis narrow band light source corresponding to the superficial blood vessels 68s (superficial region 70s), and the second laser light emitted from laser light source 44-2 (laser 2) is non-emphasis narrow band light corresponding to the superficial blood vessels 68s (superficial region 70s). The wavelength of the second laser light, the non-emphasis narrow band light corresponding to the superficial blood vessels 68s, is 445 nm and is included in the blue range 58B, as shown in FIG. 14. In FIG. 14, the ordinate axis of the laser light spectrum is drawn in an arbitrary scale.

Laser light source 44-4 (laser 4) is a non-emphasis narrow band light source corresponding to the intermediate blood vessels 68m (intermediate region 70m), and the fourth laser light emitted from laser light source 44-4 (laser 4) is non-emphasis narrow band light corresponding to the intermediate blood vessels 68m (intermediate region 70m). The wavelength of the fourth laser light, the non-emphasis narrow band light corresponding to the intermediate blood vessels 68m, is 515 nm and is included in the green range 58G, as shown in FIG. 14.

Laser light source 44-5 (laser 5) is an emphasis narrow band light source corresponding to the deep blood vessels 68d (deep region 70d), and the fifth laser light emitted from laser light source 44-5 (laser 5) is emphasis narrow band light corresponding to the deep blood vessels 68d (deep region 70d). The wavelength of the fifth laser light, the emphasis narrow band light corresponding to the deep blood vessels 68d, is 595 nm and is included in the red range 58R, as shown in FIG. 14.

After being guided by the optical fibers 48-2, 48-4, and 48-5, rays of the second laser light, fourth laser light, and fifth laser light are combined together by the light combiner 50.

The combined ray of the second laser light, fourth laser light, and fifth laser light is converted into light having a desirable light distribution by the light converter 54 at the distal end of the insertion section 26, and the resultant light is radiated to the observation object O as illumination light IL.

The second laser light whose wavelength is included in the blue range 58B has a reach length up to the superficial region 70s. Where the second laser light is radiated to the observation object O, a small light intensity difference occurs between the light intensity that the reflected and scattered light RL has in the superficial blood vessels 68s and the light intensity that the reflected and scattered light RL has near the superficial blood vessels 68s. In other words, a low contrast is provided for the superficial blood vessels 68s. That is, the superficial blood vessels 68s are not emphasized.

The reflected and scattered light RL of the illumination light IL in the observation object O is detected by the imager 22. The imager 22 detects the reflected and scattered light RL of the second laser light whose wavelength is included in the blue range 58B with the B light detection element, so as to generate a B imaging signal. The B imaging signal is output to the image processor 24. The image processor 24 performs image processing for the B imaging signal output from the imager 22 in accordance with observation mode information, so as to generate a B image signal. In the deep blood vessel emphasis mode, the B image signal generated from the B imaging signal acquired with the second laser light, non-emphasis narrow band light, is a non-emphasis image signal.

The fourth laser light whose wavelength is included in the green range 58G has a reach length up to the intermediate region 70m. Where the fourth laser light is radiated to the observation object O, a small light intensity difference occurs between the light intensity that the reflected and scattered light RL has in the intermediate blood vessels 68m and the light intensity that the reflected and scattered light RL has near the intermediate blood vessels 68m. In other words, a low contrast is provided for the intermediate blood vessels 68m. That is, the intermediate blood vessels 68m are not emphasized.

The imager 22 detects the reflected and scattered light RL of the fourth laser light whose wavelength is included in the green range 58G with the G light detection element, so as to generate a G imaging signal. The G imaging signal is output to the image processor 24. The image processor 24 performs image processing for the G imaging signal output from the imager 22 in accordance with observation mode information, so as to generate a G image signal. In the deep blood vessel emphasis mode, the G image signal generated from the G imaging signal acquired with the fourth laser light, non-emphasis narrow band light, is a non-emphasis image signal.

The fifth laser light whose wavelength is included in the red range 58R has a reach length up to the deep region 70d. Where the fifth laser light is radiated to the observation object O, a large light intensity difference occurs between the light intensity that the reflected and scattered light RL has in the deep blood vessels 68d and the light intensity that the reflected and scattered light RL has near the deep blood vessels 68d. In other words, a high contrast is provided for the deep blood vessels 68d. That is, the deep blood vessels 68d are emphasized.

The imager 22 detects the reflected and scattered light RL of the fifth laser light whose wavelength is included in the red range 58R with the R light detection element, so as to generate an R imaging signal. The R imaging signal is output to the image processor 24. The image processor 24 performs image processing for the R imaging signal output from the imager 22 in accordance with observation mode information, so as to generate an R image signal. In the deep blood vessel emphasis mode, the R image signal generated from the R imaging signal acquired with the fifth laser light, emphasis narrow band light, is an emphasis image signal.

That is, the light intensity that the reflected and scattered light RL of the illumination light IL has in the deep blood vessels 68d has a larger intensity difference with respect to the light intensity that the reflected and scattered light RL has near the blood vessels (in the mucous membrane or the like) than that of the light intensity that the reflected and scattered light RL has in the superficial blood vessels 68s and the intermediate blood vessels 68m.

In the illumination light IL in the intermediate blood vessel emphasis mode, the deep region 70d is an attention depth region, and the superficial region 70s and the intermediate region 70m are non-attention depth regions.

This observation mode is effective in observing the deep blood vessels 68d in detail.

The image processor 24 performs at least one of the contrast emphasis image process, outline (edge) emphasis image process, and blood vessel structure image process for the imaging signal (the R imaging signal in this case) that is one of the B imaging signal, G imaging signal, and R imaging signal and that corresponds to the color range including the emphasis narrow band light. The image processor 24 performs at least one of the contrast suppression image process, outline (edge) suppression image process, and blood vessel structure suppression image process for the imaging signals (the B imaging signal and the G imaging signal in this case) that are part of the B imaging signal, G imaging signal, and R imaging signal and that correspond to the color range not including the emphasis narrow band light.

The emphasis image signal and non-emphasis image signal generated by the image processor 24 are transmitted to the image display 16 to be displayed as an observation object image 78, as shown in FIG. 15. That is, in this observation object image 78, the deep blood vessel image 80d showing the deep blood vessels 68d is highlighted, while the superficial blood vessel image 80s and the intermediate blood vessel image 80m showing the superficial blood vessels 68s and intermediate blood vessels 68m are not highlighted.

<Normal Observation Mode>

Where the user enters the normal observation mode from the input device 18 as an observation mode, the input device 18 outputs observation mode information on the normal observation mode to the light source driver 46 and the image processor 24.

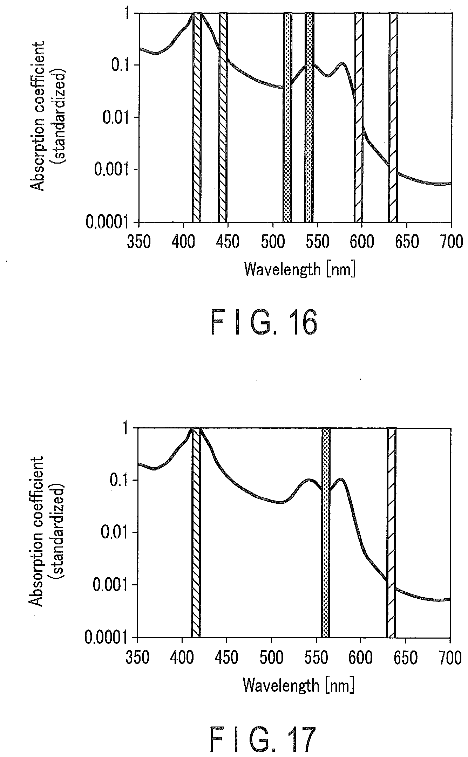

Upon receipt of the observation mode information on the deep blood vessel emphasis mode, the light source driver 46 turns on all laser light sources 44-1 to 44-6, so as to cause the laser light sources 44-1 to 44-6 to emit first laser light to sixth laser light. FIG. 16 shows a laser light spectrum exhibited then (the ordinate axis of the laser light spectrum is drawn in an arbitrary scale).

In the normal observation mode, the light quantity ratio among the laser light sources 44-1 to 44-6 is determined so that the illumination light IL has high color rendering property or high color reproduction property. For example, the color of broadband illumination light IL, such as xenon lamp or halogen lamp, is reproduced. Alternatively, the color of observation object O irradiated with broadband illumination light IL, such as xenon lamp or halogen lamp, is reproduced.

In the normal observation mode, more laser light sources are turns on than in the blood vessel emphasis modes, in order to enhance the color rendering property or color reproduction property.

As described above, the endoscope apparatus 10 according to the first embodiment of the present invention includes an imager 22 that detects reflected and scattered light RL of illumination light IL radiated to an observation object O to output an imaging signal, and an image processor 24 that generates an image signal from the imaging signal, the image processor 24 generating an emphasis image signal corresponding to narrow band light included in an emphasis wavelength range that includes, for an optical absorption spectrum of a diagnosis target substance present in the observation object, at least one of at least a maximum wavelength that takes at least a maximum value and a color-range largest wavelength that takes a color-range largest value that is a largest value of the optical absorption spectrum in any one color range of three color ranges, a first color range, a second color range, and a third color range, and a non-emphasis image signal corresponding to narrow band light included in a non-emphasis wavelength range that is a wavelength range that does not include an emphasis wavelength range.

As can be seen, an emphasis image signal corresponding to an emphasis wavelength range that includes either an optical absorption maximum wavelength of a diagnosis target substance or a color-range largest wavelength and a non-emphasis image signal corresponding to a non-emphasis wavelength range that does not include an emphasis wavelength range are generated, so that, by displaying the emphasis image signal and the non-emphasis image signal, a diagnosis target substance located in a specific depth region can be highlighted relative to the other depth regions.

The endoscope apparatus 10 according to the first embodiment of the present invention further includes an illuminator 20 that radiates illumination light IL including rays of narrow band light having mutually different peak wavelengths or central wavelengths, the rays of narrow band light included in the illumination light IL include at least a ray of emphasis narrow band light including a peak wavelength or a central wavelength in an emphasis wavelength range and at least a ray of non-emphasis narrow band light including a peak wavelength or a central wavelength in a non-emphasis wavelength range, and the image processor 24 generates an emphasis image signal from an imaging signal corresponding to emphasis narrow band light and a non-emphasis image signal from an imaging signal corresponding to non-emphasis narrow band light.

As described above, at least a ray of emphasis narrow band light included in an emphasis wavelength range including an light absorption maximum wavelength of a diagnosis target subject or a color-range largest value and at least a ray of non-emphasis narrow band light included in a non-emphasis wavelength range that does not include an emphasis wavelength range are used, so that the image processor 24 can generate an emphasis image signal from an imaging signal corresponding to emphasis narrow band light and a non-emphasis image signal from an imaging signal corresponding to non-emphasis narrow band light.

Emphasis narrow band light has a reach length up to an attention depth region of an observation object O and non-emphasis narrow band light has a reach length up to a non-attention depth region of the observation object O different from the attention depth region. Reflected and scattered light RL of the illumination light IL present in the diagnosis target substance located in the attention depth region has a larger light intensity difference with respect to reflected and scattered light RL located near the diagnosis target substance than that of reflected and scattered light RL of illumination light IL present in a diagnosis target substance located in the non-attention depth region.

Therefore, the diagnosis target substance located in the attention depth region has a high contrast and can be emphasized more than the diagnosis target substance located in the non-attention region.

The illuminator 20 has laser light sources 44-1 to 44-6, which are narrow band light sources that emit narrow band light, and a light source driver 46 that controls the narrow band light sources. The narrow band light sources includes at least an emphasis narrow band light source that emits emphasis narrow band light and at least a non-emphasis narrow band light source that emits non-emphasis narrow band light, regarding, as the attention depth region, at least one of a first depth region, a second depth region deeper than the first depth region, and a third depth region deeper than the first and second depth regions.

The light source driver 46 switches between emphasis narrow band light sources and non-emphasis narrow band light sources in accordance with which of the first, second and third depth region is regarded as the attention depth region, and can emphasize a diagnosis target substance present in the attention depth region.

At least a ray of the non-emphasis narrow band light is included in a color range that is one of the three color ranges and that does not include emphasis narrow band light.

Accordingly, the non-emphasis narrow band light supplements the color range and enhances the color reproduction property.

The attention depth region is the first depth region, the non-attention depth region includes at least either of the second depth region and the third depth region, and at least a ray of non-emphasis narrow band light has a longer wavelength than that of emphasis narrow band light.

Accordingly, the diagnosis target substance present in the first depth range can be emphasized.

In this case, the first depth region is the superficial region 70s of the observation object O, the first color range is the blue range 58B, the second color range is the green range 58G, the third color range is the red range 58R, the emphasis narrow band light is included at least in the blue range 58B, and the non-emphasis narrow band light is included in at least either of the green range 58G and the red range 58R.

Accordingly, the diagnosis target substance present in the superficial region 70s of the observation object O can be emphasized.

Alternatively, the attention depth region may be the second depth region; the non-attention depth region includes at least either of the first depth region and the second depth region, and at least a ray of non-emphasis narrow band light has a shorter or longer wavelength than that of emphasis narrow band light.

Accordingly, the diagnosis target substance present in the second depth region can be emphasized.

In this case, the second depth region is the intermediate region 70m of the observation object O, which is deeper than the superficial region 70s and shallower than the deep region 70d, the first color range is the blue range 58B, the second color range is the green range 58G, the third color range is the red range 58R, the emphasis narrow band light is included at least in the green range 58G, and the non-emphasis narrow band light is included in at least either of the blue range 58B and the green range 58G.

Accordingly, the diagnosis target substance present in the intermediate region 70m of the observation object O can be emphasized.

Alternatively, the attention depth region may be the third depth region; the non-attention depth region includes at least either of the first depth region and the second depth region, and at least a ray of non-emphasis narrow band light has a shorter wavelength than that of emphasis narrow band light.

Accordingly, the diagnosis target substance present in the third depth region can be emphasized.

In this case, the third depth region is the deep region 70d of the observation object O, the first color range is the blue range 58B, the second color range is the green range 58G, the third color range is the red range 58R, the emphasis narrow band light is included in the red range 58R, and the non-emphasis narrow band light is included in at least either of the blue range 58B and the green range 58G.

Accordingly, the diagnosis target substance present in the deep region 70d of the observation object O can be emphasized.

Either the emphasis narrow band light or the non-emphasis narrow band light is included in each of the three color ranges.

Owing to this, the illumination light IL has three rays of narrow band light included in the three color ranges, and thus enables observation with enhanced color reproducibility.

In this case, the emphasis narrow band light and the non-emphasis narrow band light are set to the intensity ratio such that the illumination light IL can be white light.

Accordingly, observation is enabled with enhanced color reproducibility.

All of the emphasis narrow band light mentioned above is included in one of the three color ranges.

Accordingly, only the diagnosis target substance present in the attention depth region can be emphasized.

In this case, single emphasis narrow band light is used.

Accordingly, only the diagnosis target substance present in the attention depth region can be emphasized.

Non-emphasis narrow band light is not included in the color range that includes the emphasis narrow band light.

Accordingly, only the diagnosis target substance present in the attention depth region can be emphasized.

Emphasis narrow band light and non-emphasis narrow band light in a color range adjacent to the color range including the emphasis narrow band light are emitted sequentially at different timings, and the imager 22 outputs different imaging signals corresponding to them.

Owing to this, a contrast decrease resulting from the mixing of the emphasis narrow band light and the non-emphasis narrow band light is prevented, so that the diagnosis target substance present in the attention depth region can be emphasized effectively.

The imager 22 outputs a first imaging signal, a second imaging signal, and a third imaging signal in response to the reception of reflected and scattered light RL included in each of the three color ranges, and the image processor 24 performs at least one of the contrast emphasis image process, edge emphasis image process, and blood vessel structure image process for the imaging signal that is one of the first to third imaging signals and that corresponds to the color range including the emphasis narrow band light.

By performing this image processing, the diagnosis target substance present in the attention depth region can be emphasized more effectively.

Alternatively, the imager 22 may output a first imaging signal, a second imaging signal, and a third imaging signal in response to the reception of reflected and scattered light RL included in each of the three color ranges, and the image processor 24 performs at least one of the contrast suppression image process, edge suppression image process, and blood vessel structure suppression image process for the imaging signal that corresponds to the color range not including the emphasis narrow band light.

By performing this image processing, the emphasis of the diagnosis target substance present in a depth region other than the attention depth region can be suppressed, and the diagnosis target substance present in the attention depth region can be emphasized more effectively.

The emphasis wavelength range is a wavelength range that is within $\pm$ 20 nm for at least one of the maximum wavelength and the color-range largest wavelength.

The emphasis wavelength range should preferably be such a wavelength range because the light absorption is great.

Alternatively, the emphasis wavelength range may be a wavelength range that is a color range in which a maximum value or a color-range largest value exists and that has values equal to or more than 1/2 of the maximum value or color-range largest value.

The emphasis wavelength range should preferably be such a wavelength range because the absorption is great.

The non-emphasis wavelength range includes at least one of a minimum wavelength that takes at least a minimum value in the optical absorption spectrum of the diagnosis target substance and a color-range smallest wavelength that takes a smallest value in any of the three color ranges.

The non-emphasis wavelength range should preferably be such a wavelength range because the absorption is small.

In this case, the non-emphasis wavelength range is a wavelength range that is within $\pm$ 20 nm for at least one of the minimum wavelength and the color-range smallest wavelength.

The non-emphasis wavelength range should preferably be such a wavelength range because the light absorption is small.

Alternatively, the non-emphasis wavelength range may be a wavelength range that is a color range in which a minimum value or a color-range smallest value exists and that has values equal to or less than 1.5 times of at least one of the minimum value and color-range smallest value.

The non-emphasis wavelength range should preferably be such a wavelength range because the absorption is small.

Alternatively, the non-emphasis wavelength range may be a wavelength range that is a color range in which a maximum value or a color-range largest value exists and that has values equal to or less than 1/2 of at least one of the maximum value and color-range largest value.

The non-emphasis wavelength range should preferably be such a wavelength range because the absorption is small.

The observation object O is a living tissue, and the diagnosis target substance is hemoglobin contained in the observation object O.

Owing to this, the blood vessels present in the living tissue can be emphasized.

In this case, the peak wavelength of at least a ray of emphasis narrow band light is in the wavelength range from 395 to 435 nm.

Owing to this, the superficial blood vessels 68s can be emphasized.

Alternatively, the peak wavelength of at least a ray of emphasis narrow band light may be in either the wavelength range from 520 to 560 nm or the wavelength range from 560 to 595 nm.

Owing to this, the intermediate blood vessels 68m or the deep blood vessels 68d can be emphasized.

The rays of narrow band light are rays of narrow band light having a wavelength width of 50 nm or less.

Owing to this, LEDs can be employed as the narrow band light sources.

Alternatively, the rays of narrow band light may be rays of ultra-narrow band light having a wavelength width of 5 nm or less.

Owing to this, laser light sources can be employed as the narrow band light sources.

The first color range is a blue wavelength range from 380 to 510 nm, the second color range is a green wavelength range from 490 to 610 nm, and the third color range is a red wavelength range from 590 to 780 nm.

By this wavelength setting, illumination light IL having color reproducibility can be generated.

The first color range is a blue range 58B, the second color range is a green range 58G, the third color range is a red range 48R, and the endoscope apparatus 10 has at least one of observation modes that are: a superficial diagnosis target substance emphasis mode in which the attention depth region is the first depth region, the non-attention depth region includes at least either the second depth region or the third depth region, the emphasis narrow band light is included in at least the blue range 58B, and the non-emphasis narrow band light is included in either the green range 58G or the red range 58R; an intermediate diagnosis target substance emphasis mode in which the attention depth region is the second depth region, the non-attention depth region includes at least either the first depth region or the third depth region, the emphasis narrow band light is included in at least the green range 58G, and the non-emphasis narrow band light is included in either the blue range 58B or the green range 58G; and a deep diagnosis target substance emphasis mode in which the attention depth region is the third depth region, the non-attention depth region includes at least either the first depth region or the second depth region, the emphasis narrow band light is included in at least the red range 58R, and the non-emphasis narrow band is included in either the blue range 58B or the green range 58G.

By having at least one of the superficial, intermediate and deep diagnosis target substance emphasis modes, the diagnosis target substance present in the superficial layer, intermediate layer, and deep layer of the observation object O can be observed while switching emphases.

In this case, the endoscope apparatus 10 further comprises an input device 18 through which an observation mode is entered, and the light source driver 46 controls a combination of narrow band light sources to be turned on, in accordance with the observation mode entered from the input device 18.

Accordingly, the combination of narrow band light sources to be turned on can be controlled in accordance with the entered observation mode.

[Modification 1]

Examples of how the illumination light spectrum can be modified in the superficial blood vessel emphasis mode are shown in FIGS. 17 to 20.

The wavelength of the fourth laser light, the non-emphasis narrow band light corresponding to the intermediate blood vessels 68m, need not be 515 nm but may be 560 nm (a minimum value in the green range 58G), as shown in FIG. 17.

The total number of rays of the emphasis narrow band light and non-emphasis narrow band light included in the illumination light IL may be four or more. Where the number of rays of narrow band light is four or more, the illumination light IL has further enhanced color rendering property and color reproduction property.

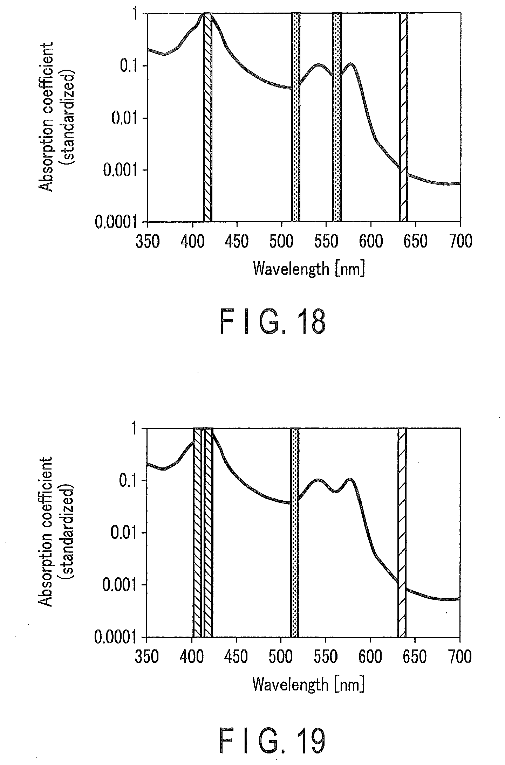

In this case, as shown in FIG. 18, two or more rays of non-emphasis narrow band light may be included in the same color range.

As shown in FIG. 19, two or more rays of emphasis narrow band light may be included in the same color range (the blue range 58B in the superficial blood vessel emphasis mode).

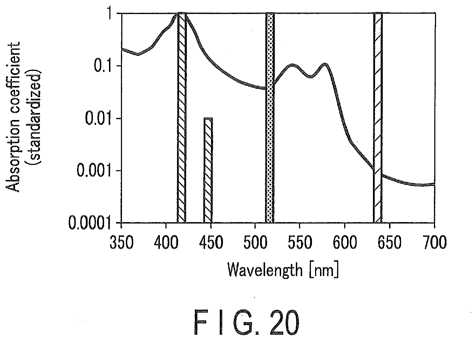

Further, as shown in FIG. 20, emphasis narrow band light and non-emphasis narrow band light may be included in the same color range. In this case, however, the emphasis narrow band light should preferably have a higher intensity than that of the non-emphasis narrow band light because the blood vessels in the attention depth region can be emphasized more.

[Modification 2]

By sequentially switching combinations of laser light sources corresponding to observation modes, observation object images 78 corresponding to the respective observation modes may be simultaneously displayed on the image display 16.

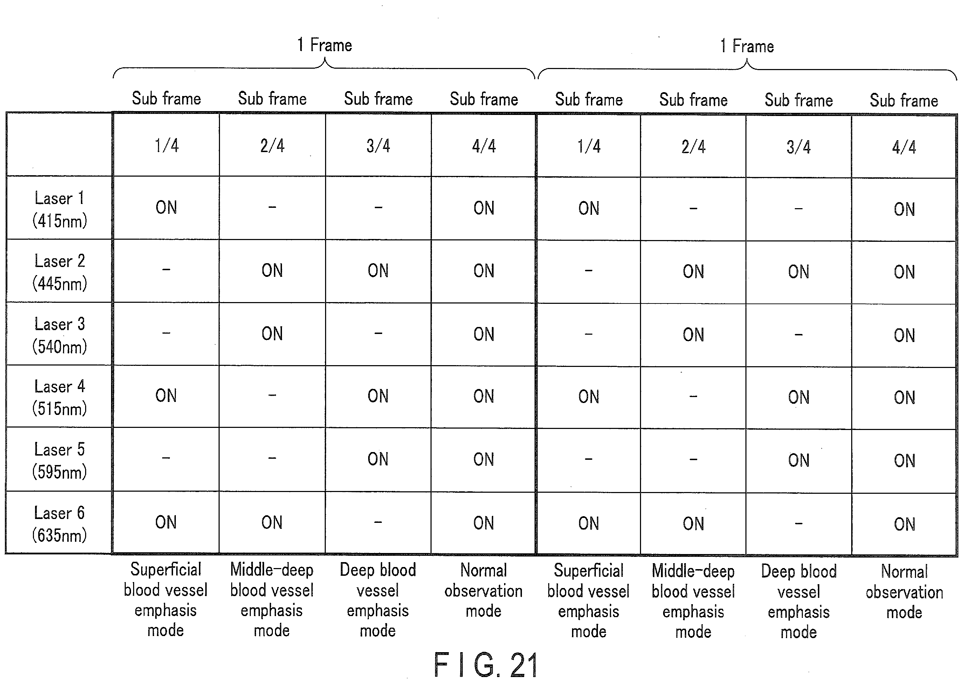

For example, one frame period, which is a general acquisition period for an imaging signal, may be divided into four sub frame periods, as shown in FIG. 21, each sub frame is made to correspond to one of observation modes, and combinations of laser sources corresponding to the observation modes are sequentially switched. That is, the storage 72 of the light source driver 46 has stored a table of laser light source lighting timings/imaging signal acquisition, such as the table shown in FIG. 21, and the light source driver 46 sequentially switches combinations of laser light sources for each sub frame, based on the table stored in the storage 72.

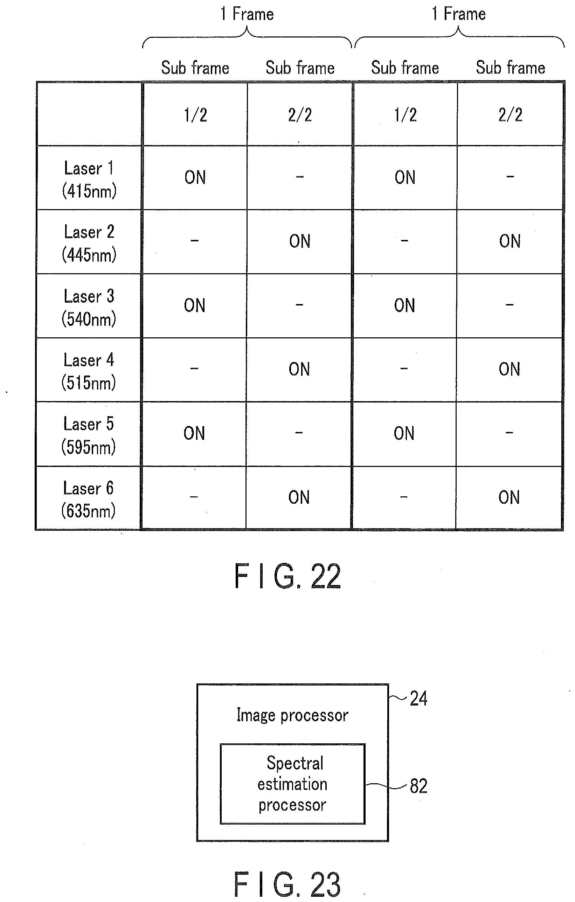

Alternatively, as shown in, for example, FIG. 22, one frame period may be divided into two sub frame periods, and three laser light sources may be turned on in each sub frame. In the first sub frame of the two sub frames, laser light source 44-1 (laser 1), laser light source 44-3 (laser 3), and laser light source 44-5 (laser 5) are turned on, and in the second sub frame, laser light source 44-2 (laser 2), laser light source 44-4 (laser 4), and laser light source 44-6 (laser 6) are turned on. In this manner, all imaging signals of laser light sources 44-1 to 44-6 are acquired in the two sub frames, and images corresponding to the respective observation modes are generated using the imaging signals. That is, the storage 72 of the light source driver 46 has stored a table of laser light source lighting timings/imaging signal acquisition, such as the table shown in FIG. 22, and the light source driver 46 sequentially switches combinations of laser light sources for each sub frame, based on the table stored in the storage 72.

The lighting timings shown in FIG. 21 and FIG. 22 are just examples, and blue, green, and red imaging signals required for image generation of observation modes may be acquired in other methods.

The number of observation modes that are sequentially switched or simultaneously displayed is not limited to four; a two or more arbitrary number of observation modes can be put into practice.

[Second Embodiment]

Next, a description will now be given of the second embodiment of the present invention. In the description below, reference will be made to how the second embodiment differs from the above-mentioned first embodiment. Same reference symbols will be used to denote same parts, and a description of such parts will be omitted.

In the first embodiment mentioned above, an observation object image 78 is generated using an image signal acquired with emphasis narrow band light as an emphasis image signal and using an image signal acquired with non-emphasis narrow band light as a non-emphasis image signal. In contrast, in the second embodiment, an emphasis image and a non-emphasis image are generated using the known spectral estimation processing.

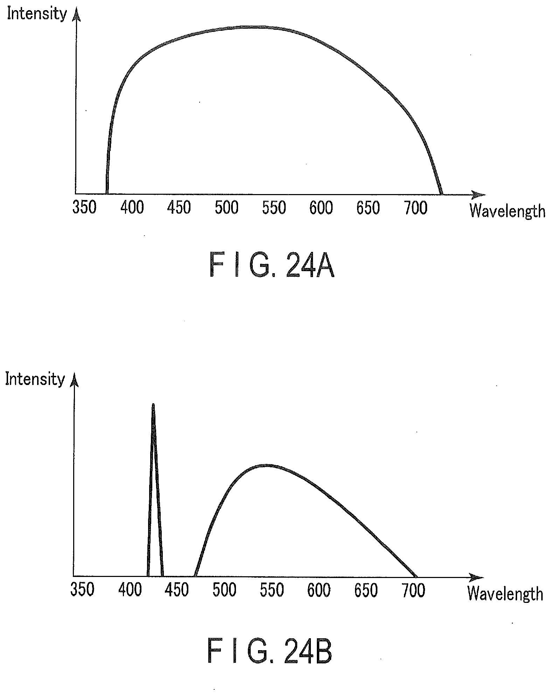

In an endoscope apparatus 10 according to the second embodiment, an image processor 24 includes a spectral estimation processor 82, as shown in FIG. 23. The illuminator 20 does not have to be provided with such narrow band light sources as described in connection with the first embodiment, and for example, the illuminator 20 may be configured to radiate illumination light IL having such broadband light as shown in FIG. 24A or FIG. 24B. That is, the illumination light IL may be light that covers virtually all ranges of the visible light wavelength ranges, as shown in FIG. 24A; alternatively, it may be a combination of narrow band light emitted from a laser light source (or an LED) and fluorescent light, as shown in FIG. 24B.

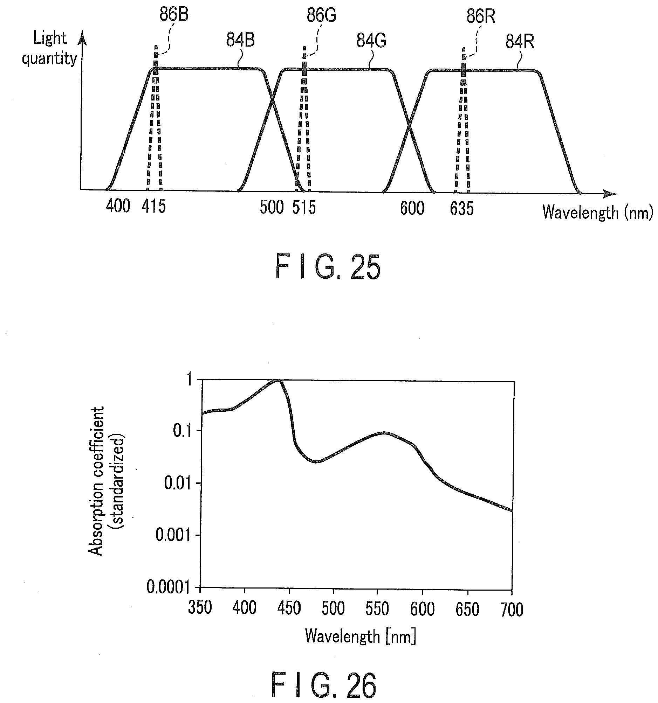

In the endoscope apparatus 10 having this structure, reflected and scattered light RL of illumination light IL including, for example, broadband light is detected by the imager 22 having color filters, and blue, green, and red imaging signals are acquired thereby. The spectral estimation processor 82 performs known spectral estimation processing for the three color imaging signals and estimates image signals obtained when emphasis narrow band light or non-emphasis narrow band light, as used in the first embodiment, is radiated. For example, when the superficial blood vessel emphasis mode is entered through the input device 18, the spectral estimation processor 82 performs the spectral estimation processing such that, as shown in FIG. 25, blue spectral estimation image signal 86B of narrow band light near wavelength 415 nm, which is an emphasis image signal, is generated from blue imaging signal 84B, green spectral estimation image signal 86G of narrow band light near wavelength 515 nm, which a non-emphasis image signal, is generated from green imaging signal 84G, and red spectral estimation image signal 86R of narrow band light near wavelength 635 nm, which an non-emphasis image signal, is generated from red imaging signal 84R.

The three imaging signals can be acquired by using a monochromatic imager 22 in place of the imager 22 having color filters. In this case, the illuminator 20 is provided with a revolving filter having three color filters whose sensitivity characteristics enable generation of imaging signals having such spectroscopic characteristics as shown in FIG. 25, three rays of illumination light are generated by switchably arranging the three color filters on the optical path in accordance with the revolving motion of the revolving filter, and return light of the rays of illumination light falls on the monochromatic imager, so that the imaging signals be acquired.

In this manner, both an emphasis image signal and a non-emphasis image signal can be generated by the spectral estimation processing. Needless to say, either of the emphasis image signal and the non-emphasis image signal may be acquired by actually radiating narrow band light, and either of them may be generated by the spectral estimation processing.

As described above, like the first embodiment, the endoscope apparatus 10 according to the second embodiment of the present invention comprises: an imager 22 that detects reflected and scattered light RL of illumination light IL radiated to an observation object O to output an imaging signal; and an image processor 24 that generates an image signal from the imaging signal, the image processor 24 generating an emphasis image signal corresponding to narrow band light included in an emphasis wavelength range that includes, for an optical absorption spectrum of a diagnosis target substance present in the observation object, at least one of a maximum wavelength that takes a maximum value and a color-range largest wavelength that takes a color-range largest value that is a largest value in any color range of three color ranges, a first color range, a second color range, and a third color range, and a non-emphasis image signal corresponding to narrow band light included in a non-emphasis wavelength range that is a wavelength range that does not include an emphasis wavelength range.

Therefore, an emphasis image signal corresponding to an emphasis wavelength range that includes either an optical absorption maximum wavelength of a diagnosis target substance or a color-range largest wavelength and a non-emphasis image signal corresponding to a non-emphasis wavelength range that does not include an emphasis wavelength range are generated, so that, by displaying the emphasis image signal and the non-emphasis image signal, only a diagnosis target substance located in a specific depth region can be highlighted.

In the endoscope apparatus 10 of the second embodiment of the present invention, the image processor 24 includes the spectral estimation processor 82 that generates at least one of the emphasis image signal and the non-emphasis image signal by performing the spectral estimation processing based on imaging signals.

As described above, the spectral estimation processor 82 can generate an emphasis image signal or a non-emphasis image signal without using emphasis narrow band light corresponding to narrow band light included in an emphasis wavelength range or non-emphasis narrow band light corresponding to narrow band light included in a non-emphasis wavelength range that is a wavelength range that does not include an emphasis wavelength range.

The illumination light may include broadband light.

Therefore, an emphasis image signal or a non-emphasis image signal can be generated using the broadband light.

[Modification 3]

In the first and second embodiments described above, the endoscope apparatus 10 has the four observation modes, which are the superficial blood vessel emphasis mode, intermediate blood vessel emphasis mode, deep blood vessel emphasis mode, and normal observation mode, but the observation modes are not limited to these.

The endoscope apparatus 10 may have only one of the superficial blood vessel emphasis mode, intermediate blood vessel emphasis mode, and deep blood vessel emphasis mode described above.

The endoscope apparatus 10 may also have another observation mode. The endoscope apparatus 10 may have a mode that radiates a normal light having a different color tone, a specific light observation mode that highlights a specific target substance in an observation object O, a fluorescence observation mode that observes fluorescent light generated when an observation object O or pharmacological agent is irradiated with excitation light.

[Modification 4]

In the first and second embodiments, the diagnosis target substance is oxyhemoglobin, but may be another substance.

For example, the diagnosis target substance may be reduced hemoglobin, which has such an absorption spectrum as shown in FIG. 26.

The diagnosis target substance may be blood in which oxyhemoglobin and reduced hemoglobin are mixed with each other. In this case, the absorption spectrum is a spectrum obtained by multiplying the mixture ratio of the oxyhemoglobin and reduced hemoglobin.

Other than hemoglobin, the diagnosis target substance may be, for example, a known autofluorescent substance, a fluorescent pharmacological agent, or a substance contained in a living body, such as the fat, bilirubin or sugar.

The present invention has been described based on the embodiments, but the present invention is in no way limited to the embodiments mentioned above. Needless to say, the present invention can be modified in various manners, without departing from the spirit and scope of the invention.

REFERENCE SIGNS LIST

10 ··· Endoscope Apparatus, 12 ··· Endoscope, 14 ··· Main Body (Video Processor), 16 ··· Image Display (Monitor), 18 ··· Input Device, 20 ··· Illuminator, 22 ··· Imager, 24 ··· Image Processor, 26 ··· Insertion Section, 28 ··· Handling Section, 30 ··· Distal End Hard Section, 32 ··· Bendable Section, 34 ··· Flexible Tube Section, 36 ··· Bending Operation Section, 38 ··· Main Body (Scope), 40 ··· Grip Section, 42 ··· Universal Cord, 44-1 to 44-6 ··· Laser Light Source, 46 ··· Light Source Driver, 48-1 to 48-6, 52 ··· Optical Fiber, 50 ··· Light Combiner, 54 ··· Light Converter, 56B ··· Spectroscopic Characteristics of Blue (B) Color Filter, 56G ··· Spectroscopic Characteristics of Green (G) Color Filter, 56R ··· Spectroscopic Characteristics of Red (R) Color Filter, 58B ··· Blue Range, 58G ··· Green Range, 58R ··· Red Range, 60B ··· Blue-Range Maximum Value, 60G ··· Green-Range Maximum Value, 62B ··· Blue-Range Largest Value, 62G ··· Green-Range Largest Value, 62R ··· Red-Range Largest Value, 64B ··· Blue-Range Minimum Value, 64G ··· Green-Range Minimum Value, 66B ... Blue-Range Smallest Value, 66G ··· Green-Range Smallest Value, 66R ··· Red-Range Smallest Value, 68d ··· Deep Blood Vessel, 68m ··· Intermediate Blood Vessel, 68s ··· Superficial Blood Vessel, 70d ··· Deep Region, 70m ··· Intermediate Region, 70s ··· Superficial Region, 72 ··· Memory, 74 ··· Defusing Member, 76 ··· Holder, 78 ··· Observation Object Image, 80d ··· Deep Blood Vessel Image, 80m ··· Intermediate Blood Vessel Image, 80s ··· Superficial Blood Vessel Image, 82 ··· Spectral Estimation Processor, 84B ··· Blue Imaging Signal, 84G ··· Green Imaging Signal, 84R ··· Red Imaging Signal, 86B ··· Blue Spectral Estimation Image Signal, 86G ··· Green Spectral Estimation Image Signal, 86R ··· Red Spectral Estimation Image Signal

**Claims**

**1.** An endoscope apparatus comprising:

an imager that detects reflected and scattered light of illumination light radiated to an observation object to output an imaging signal; and
an image processor that generates an image signal from the imaging signal,
**characterized in that**
the image processor generates
an emphasis image signal corresponding to narrow band light included in an emphasis wavelength range that includes, for an optical absorption spectrum of a diagnosis target substance present in the observation object, at least one of at least a maximum wavelength that takes at least a maximum value and a color-range largest wavelength that takes a color-range largest value that is a largest value of the optical absorption spectrum in any one color range of three color ranges, a first color range, a second color range, and a third color range, and

a non-emphasis image signal corresponding to narrow band light included in a non-emphasis wavelength range that is a wavelength range that does not include the emphasis wavelength range.

2. The endoscope apparatus according to claim 1, **characterized by** further comprising:

an illuminator that radiates illumination light including rays of narrow band light having different peak wavelengths or central wavelengths,
the rays of narrow band light included in the illumination light including

at least a ray of emphasis narrow band light having the peak wavelength or the central wavelength in the emphasis wavelength range, and
at least a ray of non-emphasis narrow band light having the peak wavelength or the central wavelength in the non-emphasis wavelength range,

the image processor generating

the emphasis image signal from the imaging signal corresponding to the emphasis narrow band light, and
the non-emphasis image signal from the imaging signal corresponding to the non-emphasis narrow band light.

3. The endoscope apparatus according to claim 2, cha racterized in that
the emphasis narrow band light has a reach length up to an attention depth region of the observation object,
the non-emphasis narrow band light has a reach length up to a non-attention depth region of the observation object different from the attention depth region, and
the reflected and scattered light of the illumination light in the diagnosis target substance present in the attention depth region has a larger light intensity difference with respect to reflected and scattered light located near the diagnosis target substance than that of reflected and scattered light of the illumination light in the diagnosis target substance present in the non-attention depth region.

4. The endoscope apparatus according to claim 2, **characterized in that**
the illuminator includes

narrow band light sources that emit rays of narrow band light, respectively, and
a light source driver that controls the narrow band light sources, respectively,

the emphasis narrow band light has a reach length up to an attention depth region of the observation object,
the non-emphasis narrow band light has a reach length up to a non-attention depth region of the observation object different from the attention depth region, and
the narrow band light sources include at least an emphasis narrow band light source that emits the emphasis narrow band light, regarding, as the attention depth region, at least one depth region of a first depth region, a second depth region deeper than the first depth region, and a third depth region deeper than the first and second depth regions, and at least a non-emphasis narrow band light source that emits the non-emphasis narrow band light.

5. The endoscope apparatus according to claim 4, **characterized in that** at least a ray of the non-emphasis narrow band light is included in a color range of the three color ranges that does not include the emphasis narrow band light.

6. The endoscope apparatus according to claim 5, **characterized in that**
the attention depth region is the first depth region,
the non-attention depth region includes at least either of the second depth region and the third depth region, and
at least a ray of the non-emphasis narrow band light has a longer wavelength than that of the emphasis narrow band light.

7. The endoscope apparatus according to claim 6, **characterized in that**
the first depth region is a superficial region of the observation object,
the first color range is a blue range, the second color range is a green range, and the third color range is a red range,
the emphasis narrow band light is included at least in the blue range, and
the non-emphasis narrow band light is included in at least either of the green range and the red range.

8. The endoscope apparatus according to claim 5, **characterized in that**
the attention depth region is the second depth region,
the non-attention depth region includes at least either of the first depth region and the third depth region, and
at least a ray of the non-emphasis narrow band light has a shorter wavelength than that of the emphasis narrow band light, or has a longer wavelength than that of the emphasis narrow band light.

9. The endoscope apparatus according to claim 8, **characterized in that**
the second depth region is an intermediate region deeper than a superficial region of the observation object and shallower than a deep region thereof,
the first color range is a blue range, the second color range is a green range, and the third color range is a red range,
the emphasis narrow band light is included at least in the green range, and
the non-emphasis narrow band light is included in at least either of the blue range and the green range.

10. The endoscope apparatus according to claim 5, **characterized in that**
the attention depth region is the third depth region,
the non-attention depth region includes at least either of the first depth region and the second depth region, and
at least a ray of the non-emphasis narrow band light has a shorter wavelength than that of the emphasis narrow band light.

11. The endoscope apparatus according to claim 10, **characterized in that**
the third depth region is a deep region of the observation object,
the first color range is a blue range, the second color range is a green range, and the third color range is a red range,
the emphasis narrow band light is included at least in the red range, and
the non-emphasis narrow band light is included in at least either of the blue range and the green range.

12. The endoscope apparatus according to claim 5, **characterized in that** one of the emphasis narrow band light and the non-emphasis narrow band light is included in each of the three color ranges.

13. The endoscope apparatus according to claim 12, **characterized in that** the emphasis narrow band light and the non-emphasis narrow band light are set to an intensity ratio such that the illumination light is white light.

14. The endoscope apparatus according to claim 13, **characterized in that**
a total number of rays of the emphasis narrow band light and non-emphasis narrow band light included in the illumination light is four or more, and
the rays of narrow band light further include the non-emphasis narrow band light that has a peak wavelength or a central wavelength different from that of any of three rays of the emphasis narrow band light and non-emphasis narrow band light included in the illumination light, so as to enhance color reproducibility of the illumination light.

15. The endoscope apparatus according to claim 5, **characterized in that** all rays of the emphasis narrow band light are included in any one of the three color ranges.

16. The endoscope apparatus according to claim 15, **characterized in that** the emphasis narrow band light is only one.

17. The endoscope apparatus according to claim 5, **characterized in that** the non-emphasis narrow band light is not included in the color range that includes the emphasis narrow band light.

18. The endoscope apparatus according to claim 5, **characterized in that** where both the emphasis narrow band light and the non-emphasis narrow band light are included in the same color range, the intensity of the emphasis narrow band light is higher than the intensity of the non-emphasis narrow band light.

19. The endoscope apparatus according to claim 5, **characterized in that** the emphasis narrow band light and the non-emphasis narrow band light of the color range adjacent to the color range including the emphasis narrow band light are emitted sequentially at different timings and are output from the imager as different imaging signals.

20. The endoscope apparatus according to claim 5, **characterized in that**
the imager receives the reflected and scattered light included in each of the three color ranges and outputs a first imaging signal, a second imaging signal, and a third imaging signal, and
the image processor performs at least one of a contrast emphasis image process, an edge emphasis image process, and a blood vessel structure image process for an imaging signal that is one of the first to third imaging signals and that corresponds to the color range including the emphasis narrow band light.

21. The endoscope apparatus according to claim 5, **characterized in that**
the imager receives the reflected and scattered light included in each of the three color ranges and outputs a first imaging signal, a second imaging signal, and a third imaging signal, and
the image processor performs at least one of a contrast suppression image process, an edge suppression image process, and a blood vessel structure suppression image process for an imaging signal that corresponds to the color range not including the emphasis narrow band light.

22. The endoscope apparatus according to claim 2, **characterized in that** the emphasis wavelength range is a wavelength range that is within ± 20 nm for at least one of the maximum wavelength and the color-range largest wavelength.

23. The endoscope apparatus according to claim 2, **characterized in that** the emphasis wavelength range is a wavelength range that is the color range in which the maximum value or the color-range largest value exists and that has values equal to or more than 1/2 of the maximum value or color-range largest value.

24. The endoscope apparatus according to claim 2, **characterized in that**
the non-emphasis wavelength range includes, for the optical absorption spectrum of the diagnosis target substance, at least one of at least a minimum wavelength that takes at least a minimum value and a color-range smallest wavelength that takes a color-range smallest value in any of the three color ranges.

25. The endoscope apparatus according to claim 24, **characterized in that** the non-emphasis wavelength range is a wavelength range that is within ± 20 nm for at least one of the minimum wavelength and the color-range smallest wavelength.

26. The endoscope apparatus according to claim 24, **characterized in that** the non-emphasis wavelength range is a color range in which the minimum value or the color-range smallest value exists and that has values equal to or less than 1.5 times of at least one of the minimum value and the color-range smallest value.

27. The endoscope apparatus according to claim 2, **characterized in that** the non-emphasis wavelength range is a color range in which the maximum value or the color-range largest value exists and that has a value less than 1/2 of at least one of the maximum value and color-range largest value.

28. The endoscope apparatus according to claim 2, **characterized in that**
the observation object is a living tissue, and
the diagnosis target substance is hemoglobin contained in the observation target.

29. The endoscope apparatus according to claim 28, **characterized in that** the peak wavelength of at least a ray of the emphasis narrow band light is in a wavelength range from 395 to 435 nm.

30. The endoscope apparatus according to claim 28, **characterized in that** the peak wavelength of at least a ray of the emphasis narrow band light is in either a wavelength range from 520 to 560 nm or a wavelength range from 560 to 595 nm.

31. The endoscope apparatus according to claim 5, **characterized in that** the rays of narrow band light are rays of narrow band light having a wavelength width of 50 nm or less.

32. The endoscope apparatus according to claim 5, **characterized in that** the rays of narrow band light are rays of ultra-narrow band light having a wavelength width of 5 nm or less.

33. The endoscope apparatus according to claim 2, **characterized in that**
the first color range is a blue wavelength range from 380 to 510 nm,
the second color range is a green wavelength range from 490 to 610 nm, and
the third color range is a red wavelength range from 590 to 780 nm.

34. The endoscope apparatus according to claim 5, **characterized in that**

the first color range is a blue range, the second color range is a green range, and the third color range is a red range, and

the endoscope apparatus has at least one of observation modes that are:

a superficial diagnosis target substance emphasis mode in which the attention depth region is the first depth region, the non-attention depth region includes at least either the second depth region or the third depth region, the emphasis narrow band light is included in at least the blue range, and the non-emphasis narrow band light is included in either the green range or the red range;

an intermediate diagnosis target substance emphasis mode in which the attention depth region is the second depth region, the non-attention depth region includes at least either the first depth region or the third depth region, the emphasis narrow band light is included in at least the green range, and the non-emphasis narrow band light is included in either the blue range or the green range; and

a deep diagnosis target substance emphasis mode in which the attention depth region is the third depth region, the non-attention depth region includes at least either the first depth region or the second depth region, the emphasis narrow band light is included in at least the red range, and the non-emphasis narrow band is included in either the blue range or the green range.

35. The endoscope according to claim 34, **characterized by** further comprising:

an input device through which the observation mode is entered,
the light source driver controlling a combination of narrow band light sources caused to emit, in accordance with the observation mode entered through the input device.

36. The endoscope apparatus according to claim 1, **characterized in that** the image processor includes a spectral estimation processor that generates at least one of the emphasis image signal and the non-emphasis image signal by performing spectral estimation processing based on the imaging signal.

37. The endoscope apparatus according to claim 36, **characterized in that** the illumination light includes broadband light.

FIG. 1

F I G. 2

F I G. 3

56B    56G    56R

FIG. 4

58R

58G

58B

60B
62B

60G
62G

62R

64G

64B
66B
64G

66G

66R

FIG. 5

FIG. 6A

Short wavelength ⟶ Long wavelength

FIG. 6B

| | Superficial blood vessel emphasis mode | Middle-deep blood vessel emphasis mode | Deep blood vessel emphasis mode | Normal observation mode |
|---|---|---|---|---|
| Laser light sources to be turned on | Laser 1 (emphasis light source for superficial blood vessels) | Laser 2 (non-emphasis light source for superficial blood vessels) | Laser 2 (non-emphasis light source for superficial blood vessels) | All laser light sources |
| | Laser 4 (non-emphasis light source for middle-deep blood vessels) | Laser 3 (emphasis light source for middle-deep blood vessels) | Laser 4 (non-emphasis light source for middle-deep blood vessels) | |
| | Laser 6 (non-emphasis light source for deep blood vessels) | Laser 6 (non-emphasis light source for deep blood vessels) | Laser 5 (emphasis light source for deep blood vessels) | |

FIG. 7

|  | Superficial blood vessel emphasis mode | Middle-deep blood vessel emphasis mode | Deep blood vessel emphasis mode | Normal observation mode |
|---|---|---|---|---|
| Laser light to be emitted | First laser light (emphasis light for superficial blood vessels) | Second laser light (non-emphasis light for superficial blood vessels) | Second laser light (non-emphasis light for superficial blood vessels) | All laser light |
|  | Fourth laser light (non-emphasis light for middle-deep blood vessels) | Third laser light (emphasis light for middle-deep blood vessels) | Fourth laser light (non-emphasis light for middle-deep blood vessels) |  |
|  | Sixth laser light (non-emphasis light for deep blood vessels) | Sixth laser light (non-emphasis light for deep blood vessels) | Fifth laser light (emphasis light for deep blood vessels) |  |

FIG. 8

FIG. 9

FIG. 10

F I G. 11

F I G. 12

F I G. 13

FIG. 14

FIG. 15

F I G. 16

F I G. 17

F I G. 18

F I G. 19

F I G. 20

| | 1 Frame | | | | 1 Frame | | | |
| | Sub frame | Sub frame | Sub frame | Sub frame | Sub frame | Sub frame | Sub frame | Sub frame |
|---|---|---|---|---|---|---|---|---|
| | 1/4 | 2/4 | 3/4 | 4/4 | 1/4 | 2/4 | 3/4 | 4/4 |
| Laser 1 (415nm) | ON | – | – | ON | ON | – | – | ON |
| Laser 2 (445nm) | – | ON | ON | ON | – | ON | ON | ON |
| Laser 3 (540nm) | – | ON | – | ON | – | ON | – | ON |
| Laser 4 (515nm) | ON | – | ON | ON | ON | – | ON | ON |
| Laser 5 (595nm) | – | – | ON | ON | – | – | ON | ON |
| Laser 6 (635nm) | ON | ON | – | ON | ON | ON | – | ON |
| | Superficial blood vessel emphasis mode | Middle-deep blood vessel emphasis mode | Deep blood vessel emphasis mode | Normal observation mode | Superficial blood vessel emphasis mode | Middle-deep blood vessel emphasis mode | Deep blood vessel emphasis mode | Normal observation mode |

F I G. 21

EP 3 354 188 A1

EP 3 354 188 A1

| | Sub frame | Sub frame | Sub frame | Sub frame |
|---|---|---|---|---|
| | 1 Frame | | 1 Frame | |
| | 1/2 | 2/2 | 1/2 | 2/2 |
| Laser 1 (415nm) | ON | – | ON | – |
| Laser 2 (445nm) | – | ON | – | ON |
| Laser 3 (540nm) | ON | – | ON | – |
| Laser 4 (515nm) | – | ON | – | ON |
| Laser 5 (595nm) | ON | – | ON | – |
| Laser 6 (635nm) | – | ON | – | ON |

F I G. 22

Image processor ~24

Spectral estimation processor ~82

F I G. 23

39

F I G. 24A

F I G. 24B

F I G. 25

F I G. 26

<div align="center">

**INTERNATIONAL SEARCH REPORT**

</div>

| | International application No. |
|---|---|
| | PCT/JP2015/076930 |

| A. CLASSIFICATION OF SUBJECT MATTER |
|---|
| *A61B1/00*(2006.01)i, *A61B1/06*(2006.01)i |

According to International Patent Classification (IPC) or to both national classification and IPC

| B. FIELDS SEARCHED |
|---|
| Minimum documentation searched (classification system followed by classification symbols) |
| A61B1/00-1/32, G02B23/24-23/26 |

Documentation searched other than minimum documentation to the extent that such documents are included in the fields searched

| Jitsuyo Shinan Koho | 1922-1996 | Jitsuyo Shinan Toroku Koho | 1996-2015 |
|---|---|---|---|
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII)

| C. DOCUMENTS CONSIDERED TO BE RELEVANT | | |
|---|---|---|
| Category* | Citation of document, with indication, where appropriate, of the relevant passages | Relevant to claim No. |
| X<br><br><br>Y<br><br>A | JP 2014-61152 A (Fujifilm Corp.),<br>10 April 2014 (10.04.2014),<br>paragraphs [0004], [0030]; fig. 6<br>& CN 103654687 A | 1-12,15-17,<br>19,22-26,<br>28-35<br>13-14,20-21,<br>36-37<br>18,27 |
| Y | WO 2013/145409 A1 (Olympus Medical Systems Corp.),<br>03 October 2013 (03.10.2013),<br>paragraphs [0089] to [0131]; fig. 19<br>& JP 5362149 B1 & US 2013/0293693 A1<br>paragraphs [0148] to [0199]; fig. 19<br>& EP 2700349 A1 & CN 103582445 A | 13-14,36-37 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 December 2015 (08.12.15) | 15 December 2015 (15.12.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

| International application No. |
| --- |
| PCT/JP2015/076930 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2013-202167 A  (Fujifilm Corp.),<br>07 October 2013 (07.10.2013),<br>paragraphs [0065], [0070] to [0071], [0087] to [0089]<br>(Family: none) | 20 |
| Y | WO 2015/029709 A1  (Fujifilm Corp.),<br>05 March 2015 (05.03.2015),<br>paragraphs [0055], [0074] to [0075]<br>(Family: none) | 21 |
| A | WO 2013/042395 A1  (Olympus Medical Systems Corp.),<br>28 March 2013 (28.03.2013),<br>paragraphs [0007] to [0010], [0076] to [0093]<br>& JP 5355820 B2          & US 2013/0176411 A1<br>paragraphs [0113] to [0130]<br>& EP 2687145 A1          & CN 103501681 A | 1-37 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014061152 A